(19)

Europäisches Patentamt

European Patent Office

Office européen des brevets

(11) **EP 4 394 029 A1**

(12) **EUROPEAN PATENT APPLICATION**

(43) Date of publication:
**03.07.2024 Bulletin 2024/27**

(21) Application number: **22383318.7**

(22) Date of filing: **30.12.2022**

(51) International Patent Classification (IPC):
***C12N 5/071*** (2010.01)     *A61L 27/38* (2006.01)

(52) Cooperative Patent Classification (CPC):
**C12N 5/0625; C12N 5/0629; C12N 5/0698;**
C12N 2500/14; C12N 2500/38; C12N 2501/11;
C12N 2501/33; C12N 2502/094; C12N 2502/11;
C12N 2533/54; C12N 2533/56

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Universidad Carlos III de Madrid**
**28919 Leganés - Madrid (ES)**

(72) Inventors:
• **DE ARANDA IZUZQUIZA, Gonzalo**
**28919 Leganés (ES)**

• **JORCANO NOVAL, José, Luís**
**28919 Leganés (ES)**
• **QUÍLEZ LÓPEZ, Cristina**
**28919 Leganés (ES)**
• **RISUEÑO ROJO, Ignacio**
**28919 Leganés (ES)**
• **VELASCO BAYÓN, Diego**
**28919 Leganés (ES)**
• **VALENCIA BLANCO, Leticia**
**28919 Leganés (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(54) **ORGANOTYPIC SKIN MODEL AND USES THEREOF**

(57)     The present invention relates to a method for the *in vitro* production of an organotypic skin model, wherein said skin model is able to complete skin maturation due to suitable culture media. This methodology provides with a new product that better resembles human skin, that can be cultured for long period of times, and that is suitable for in vitro testing and modelling.

**EP 4 394 029 A1**

**Description**

**TECHNICAL FIELD**

The present invention relates to the field of tissue engineering. Particularly, the present invention relates to the field of skin models.

**BACKGROUND ART**

The generation of *in vitro* cultures of human skin is based on a bilayer model of dermis and epidermis. Different methodologies have been created based on the use of different biomaterials for the generation of the dermal component, among which are the following:

• Collagen I-based matrix: the main composition of the dermal component is collagen I, which is why it is used for the generation of most *in vitro* skin models. Given the high amounts of collagen needed to generate these models and the difficulty of obtaining it from human origin, the collagen used is normally of animal origin.
• Plasma-derived fibrin matrix: fibrinogen is a molecule present in blood plasma and is responsible for forming the fibrin clot during healing. This fibrin clot serves as a temporary matrix for the cells adjacent to the wounded area to migrate and remodel and build a definitive extracellular matrix composed mainly of collagen I during the healing process. This process is reproduced *in vitro* to generate fibrin matrices for their clinical use. However, human plasma-based fibrin matrices are weak and degrade rapidly, having a half-life of around 17 days. This culture time is not enough for the cells to remodel the dermal component and synthesize collagen, which is why it is necessary to improve the mechanical properties of the matrix. To this end, different ways have been tried to complement the dermal component, increase its stability, lengthen its half-life and help in its maturation. However, none of the methods explored has fulfilled this objective.
• Exogenous-derived fibrin matrix: Given the concentration limitations offered by plasma and the variability in reproducibility depending on the donor, fibrin matrix supplemented with exogenous fibrinogen as a source of fibrin appears as an alternative. However, the skin models generated with exogenous fibrinogen present high percentages of contraction and consequent instability. For this reason, these cultures are generated with high concentrations of fibrinogen, being negative in the correct development of the skin.

In view of the above, the most important limitations faced at the time of producing suitable *in vitro* skin models are: 1) instability of the model with accelerated degradation, which reduces cultivation times; 2) the high levels of fibrinogen and collagen used to avoid matrix contraction do not favour the correct maturation of the cultures; 3) the use of pure fibrinogen is not an optimal material for the generation of organotypic cultures, since it lacks the proteins and growth factors present in the plasma and beneficial for the correct formation of the skin; and 4) the culture conditions used in long-term cultures (around 30 days) cause loss of homeostasis, leading to a terminal differentiation of the epidermis, losing the basal layer of cells in favour of the *stratum corneum,* which causes that the obtained models do not have a functional and structurally correct skin at long cultivation times.

The above problems are solved herein by providing a matured skin model that can be cultured for long times without contracting or losing its properties, i.e., with maintained homeostasis, being highly reproducible and suitable for clinical use, cosmetic use, and drug testing and textile testing.

**SUMMARY OF INVENTION**

In a **first aspect,** the present invention relates to a method for the in vitro production of an organotypic skin model, the method comprising the steps of:

a) providing a solution comprising stromal cells, blood plasma, fibrinogen, and a source of calcium; and incubating said solution until a hydrogel is formed,
b) adding to the hydrogel of step a), preferably on its surface, a cell population comprising keratinocytes, and culturing the hydrogel in a keratinocyte culture media to allow the keratinocytes to grow until at least 90% of confluency is reached, wherein the keratinocyte culture media is characterized by having a percentage of serum of more than 8% (v/v),
c) transferring the product resulting from step b) to an air-liquid interface and culturing said product in a suitable epidermal differentiation culture media to provide an organotypic skin model with epidermal differentiation, wherein the epidermal differentiation culture media is characterized by having a percentage of serum of less than 1% (v/v), and
d) maturing the organotypic skin model with epidermal differentiation of step c) in an epidermal maintenance culture media, wherein the epidermal maintenance culture media is characterized by having a percentage of serum of between 4-6% (v/v).

Preferably, the step d) is carried out until the keratinocytes are differentiated as indicated by the expression in the epidermal layer of Keratin 5, Keratin 14, Keratin 10, Collagen IV, Laminin 5, Loricrin, Filaggrin and/or Involucrin proteins, thereby providing a mature organotypic skin model.

Preferably, the matured organotypic skin model obtained in step d) is characterized by comprising:

- an epidermal compartment, wherein the epidermal compartment is in the upper part of the organotypic skin model, and it comprises keratinocytes and hyaluronic acid,
- a basement membrane, wherein said basement membrane is located below the epidermal compartment, and where it is characterized by being a membrane comprising collagen IV and laminin 5,
- a dermal compartment, wherein the dermal compartment is located in the base of the organotypic skin culture, and it comprises fibroblasts, collagen III and I.

Preferably, the epidermal compartment is characterized by comprising, from top to bottom:

- a cornified epidermal layer or stratum corneum comprising cells, preferably corneocytes, and filaggrin,
- a granular epidermal layer comprising cells, preferably keratinocytes, expressing loricrin,
- a suprabasal (or spinous) epidermal layer comprising cells, preferably keratinocytes, expressing keratin 10 and involucrin, and
- a basal epidermal layer comprising cells, preferably keratinocytes, ex-pressing keratin 5 and 14.

Preferably, the cell population comprising fibroblasts cells added in step a) are dermal fibroblasts from human foreskin. Preferably, the cell population comprising keratinocytes cells added in step b) are epidermal keratinocytes from human foreskin. Preferably, the total number of fibroblasts cells added in step a) is 5.000-80.000 cells/ml, preferably 20.000 cells/ml. Preferably, the total number of keratinocytes cells added in step b) is 12.000-1.500.000 cells/ml, preferably $1 \times 10^6$ cells/ml.

Preferably, the final concentration of fibrin in the resulting hydrogel of a) is between 1.2-4.5 mg/mL, preferably 3.5 mg/mL. Preferably, the solution of plasma has a concentration of platelets of less than $10 \times 10^5$ platelets/$\mu$L. Preferably, an antifibrinolytic agent is further added in step a). Preferably, the source of calcium added in step a) is at a final concentration of between 0.01-2%, preferably 0.08% (w/v).

In a **second aspect,** the present invention provides an organotypic skin model obtained or obtainable from the method according to the first aspect or any of its embodiments.

In a **third aspect,** the present invention provides the in vitro use of an organotypic skin model obtained or obtainable from the method according to the first aspect or any of its embodiments in pharmacological development assays, chemical or pollutants toxicity assays, screening of medical and/or cosmetic drugs, testing mechanical irritations through textiles, basic study of the fundamental bi-ology of skin, and/or the processes that mediate skin ageing or skin disease.

**BRIEF DESCRIPTION OF DRAWINGS**

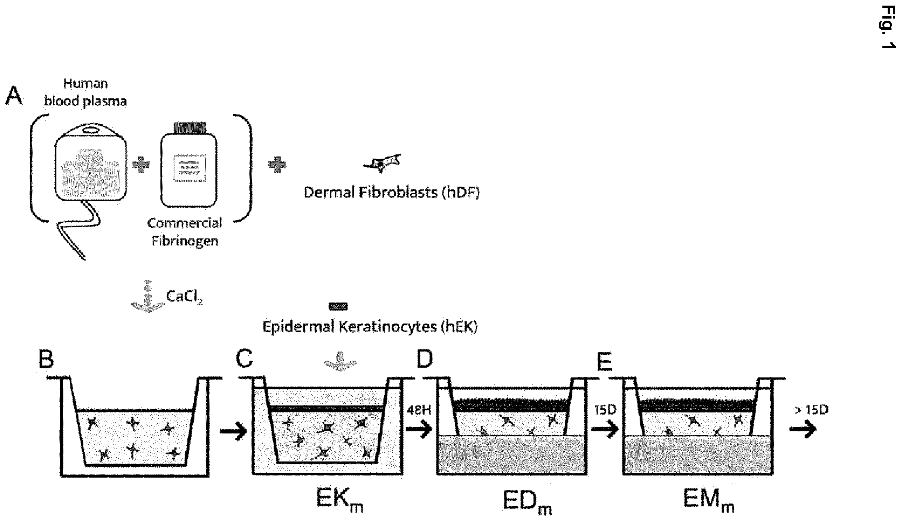

**Fig. 1:** Fabrication of organotypic skin cultures: A) Plasma-derived fibrin hydrogel preparation supplemented with commercial fibrinogen and with encapsulated fibroblasts; B) Hydrogel gelification; C) hEK seeding to form the basal epidermal layer using $EK_m$; D) Epidermal differentiation in the air liquid interface with $ED_m$ and E) Organotypic skin culture maintenance with $EM_m$.

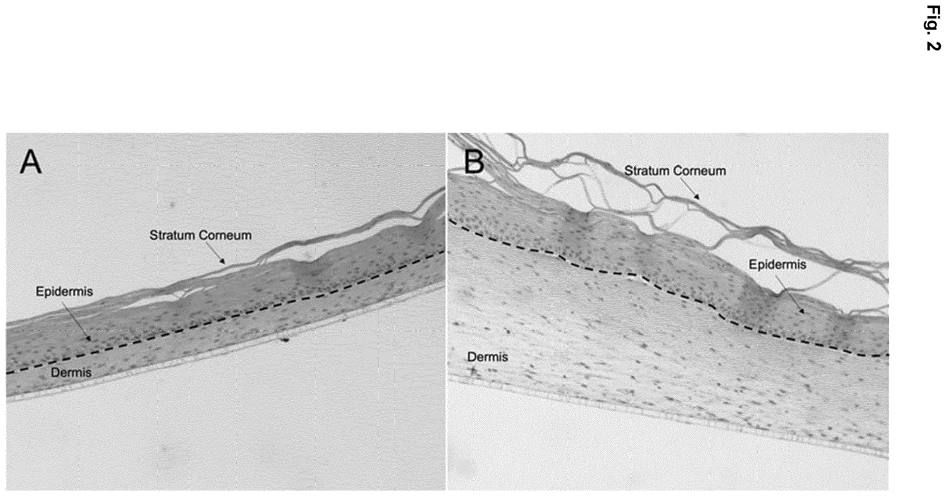

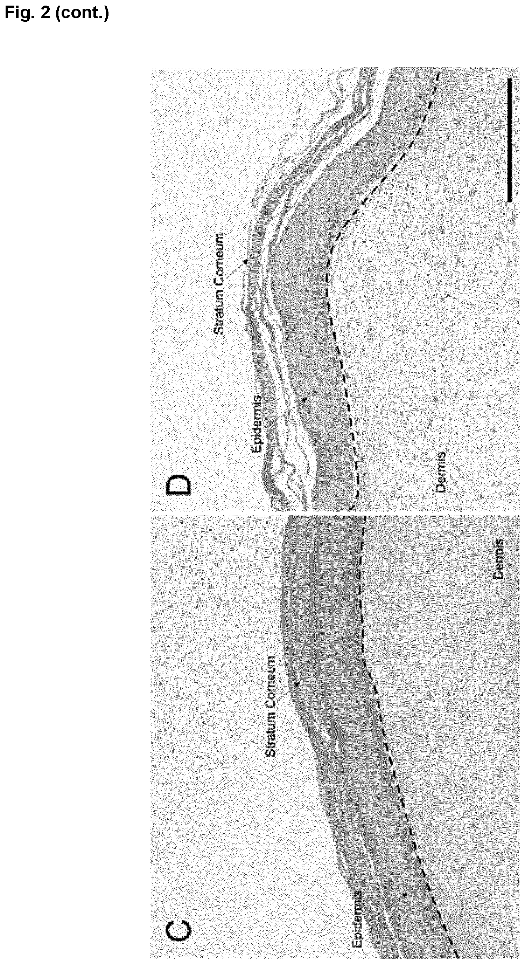

**Fig. 2:** Structural characterization of organotypic skin cultures with H/E staining for different fibrin concentrations after 15 days in culture: A) control of 1.2 mg/mL; B) 2.5mg/mL; C) 3.5mg/mL and D) 4.5 mg/mL. Scale bar: 300$\mu$m.

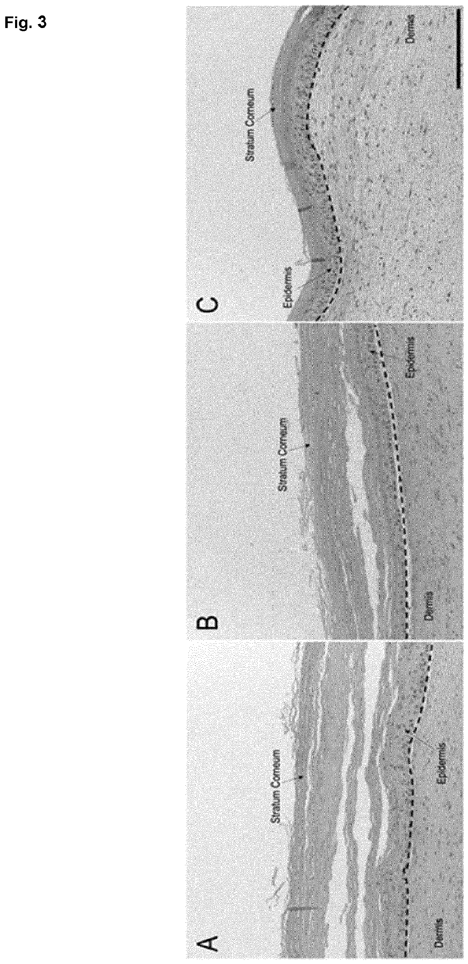

**Fig. 3:** Structural characterization of organotypic skin cultures at a final fibrin concentration of 3.5 mg/mL with H/E after 30 days in culture for different culture conditions: A) differentiation using $ED_m$; B) differentiation alternating $ED_m$ and $EK_m$ and C) differentiation using $EM_m$. Scale bar: 300$\mu$m.

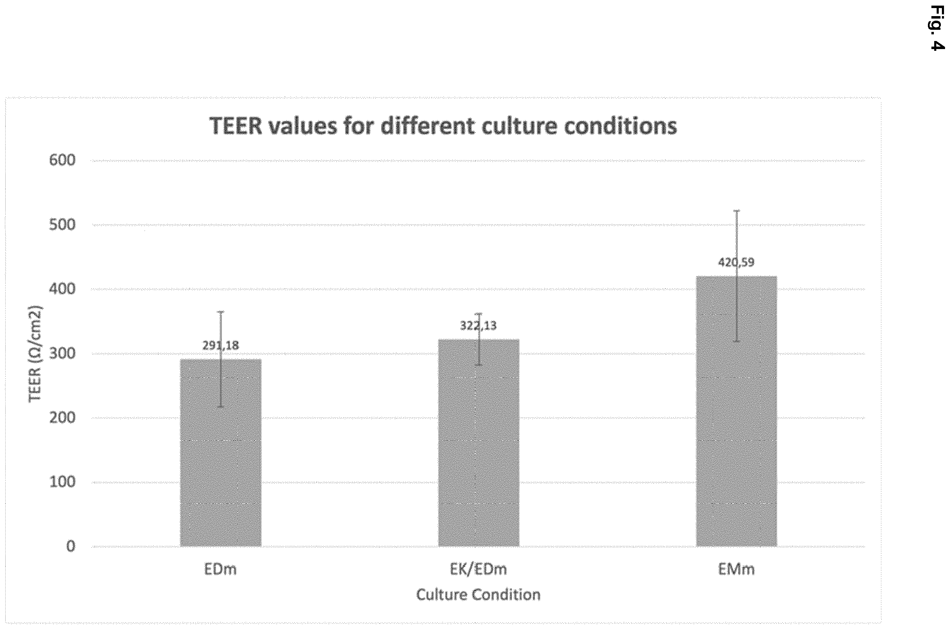

**Fig. 4:** TEER values ($\Omega \cdot cm^2$) for of organotypic skin cultures at a final fibrin concentration of 3.5 mg/mL after 30 days in culture for different culture conditions.

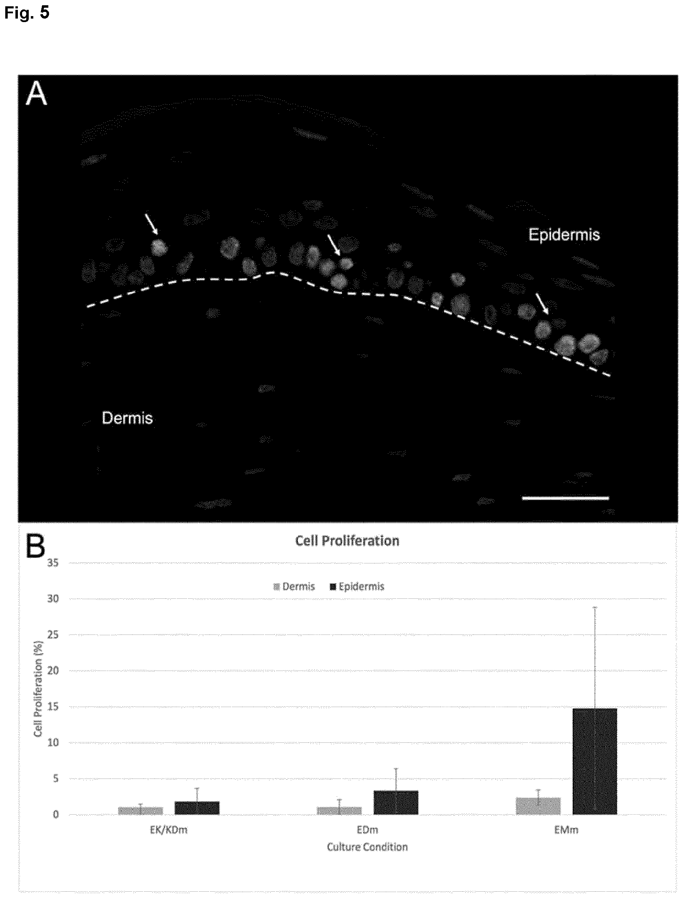

**Fig. 5:** Cell proliferation in organotypic skin cultures at a final fibrin concentration of 3.5mg/mL after 30 days in culture A) Immunofluorescence of proliferative cells after BrdU incorporation B) Cell proliferation in the dermal and epidermal compartment in terms of BrdU incorporation for different culture conditions. Scale bar: 100$\mu$m.

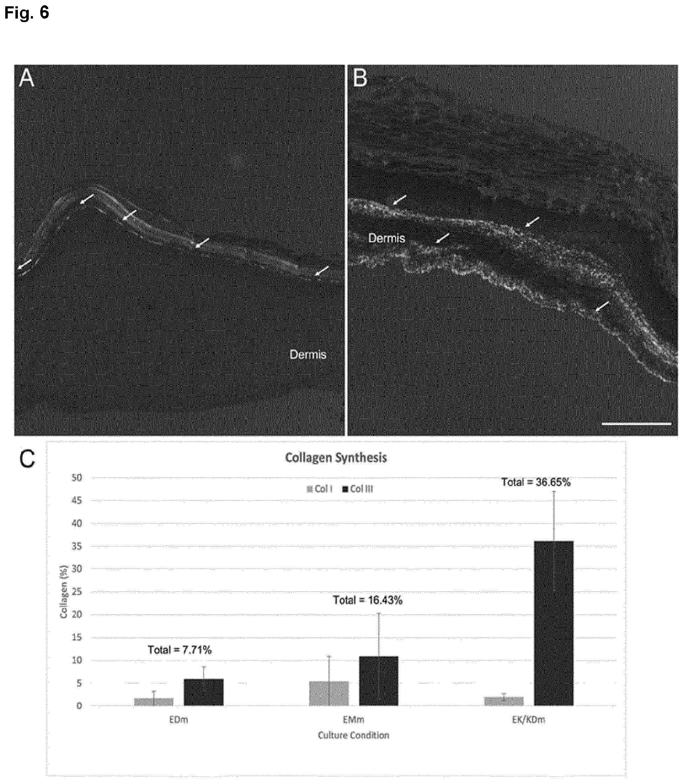

**Fig. 6:** Collagen composition of the dermal compartment using Picrosirius staining in organotypic skin cultures at a final fibrin concentration of 3.5mg/mL after A) 15 and B) 30 dais in culture. C) Collagen synthesis in organotypic skin cultures at a final fibrin concentration of 3.5mg/mL after 30 days in culture for different culture conditions. Scale bar: 300$\mu$m.

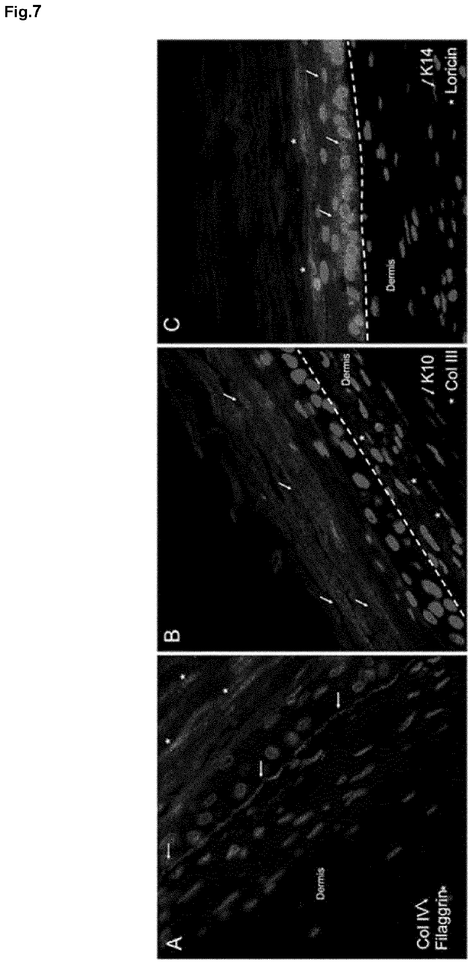

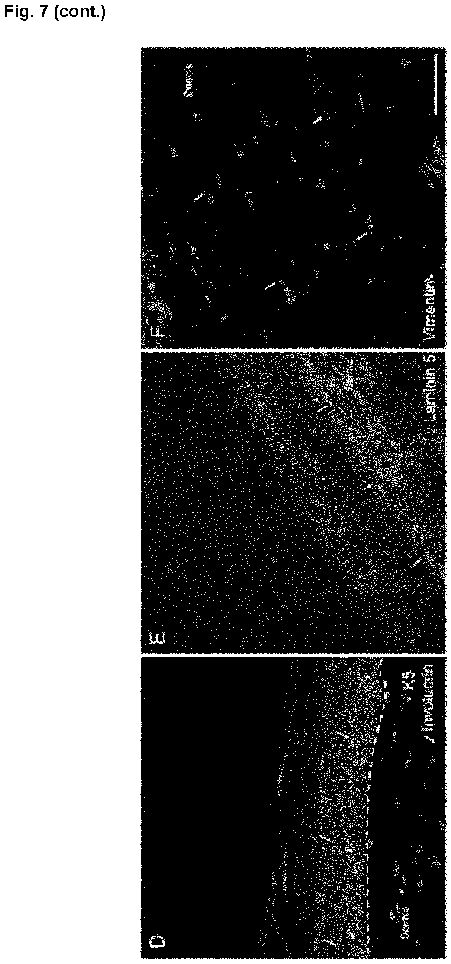

**Fig. 7:** Organotypic skin cultures at a final fibrin concentration of 3.5 mg/mL after 30 days in culture using $EM_m$ characterized through immunofluorescence staining using antibodies against dermo-epidermal markers: A) collagen IV (arrow) and Filaggrin (star); B) K10 (arrow) and collagen III (star); C) K14 (arrow) and Loricrin (star); D) Involucrin (arrow) and K5 (star); E) Laminin 5 (arrow) and F) Vimentin (arrow). Scale bar: 100$\mu$m.

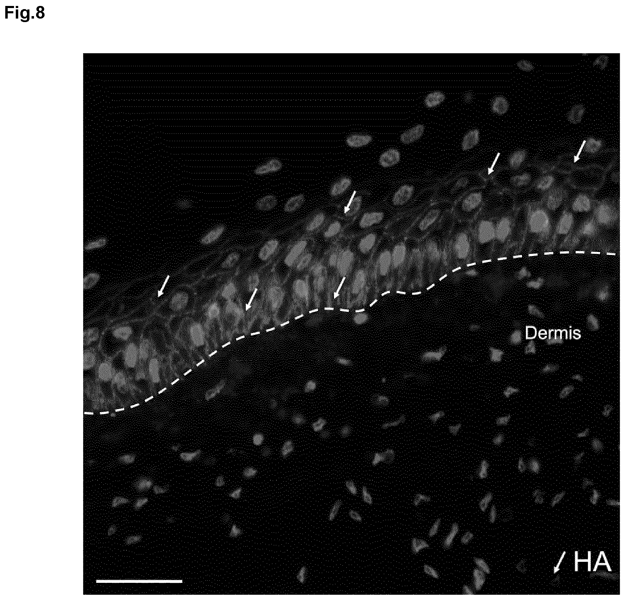

**Fig. 8:** Organotypic skin culture at a final fibrin concentration of 3.5 mg/mL after 30 days in culture using $EM_m$ characterized through immunofluorescence staining for hyaluronic acid in the intercellular space of the epidermis. Scale bar 200$\mu$m.

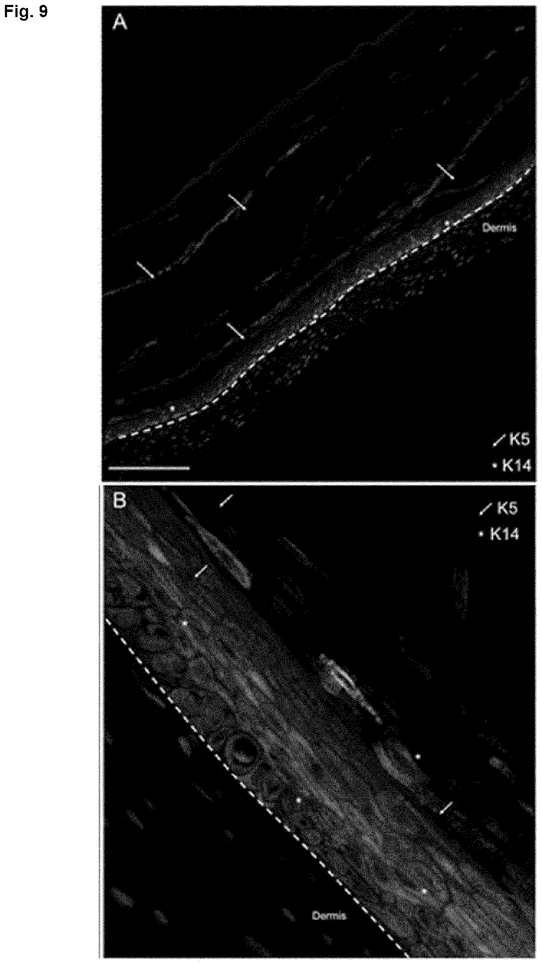

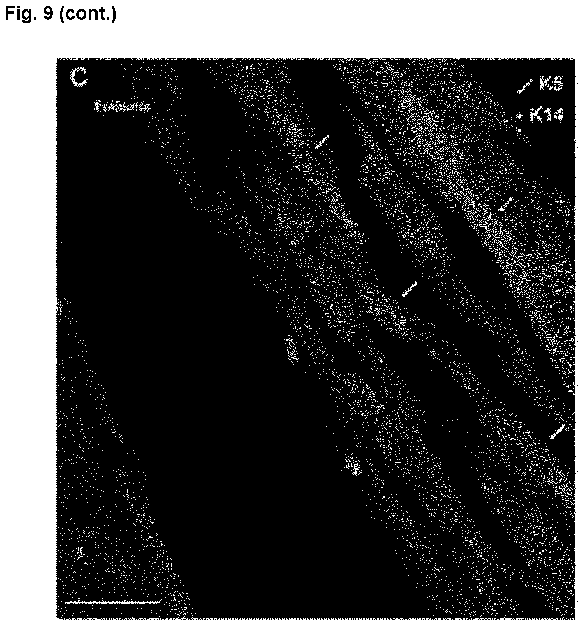

**Fig. 9:** Organotypic skin cultures at a final fibrin concentration of 3.5 mg/mL after 30 days in culture using $EK/ED_m$ characterized through immunofluorescence staining using antibodies against epidermal markers: A) Expression of K5 in the *stratum corneum.* Scale bar: 300$\mu$m. B) Co-expression of K5 and K14 in the basal and suprabasal layer and C) expression of K5 of the a-nucleated layers of the stratum *corneum* layer. Scale bar 50$\mu$m.

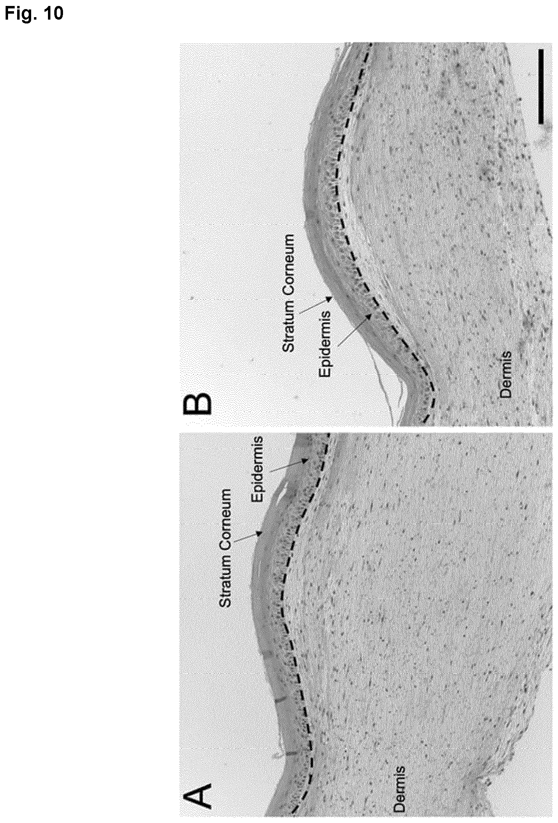

**Fig. 10:** Structural characterization of organotypic skin cultures with H/E staining after 30 days in culture in the presence (A) or in the absence (B) of antifibrinolytic agent. Scale bar 300$\mu$m.

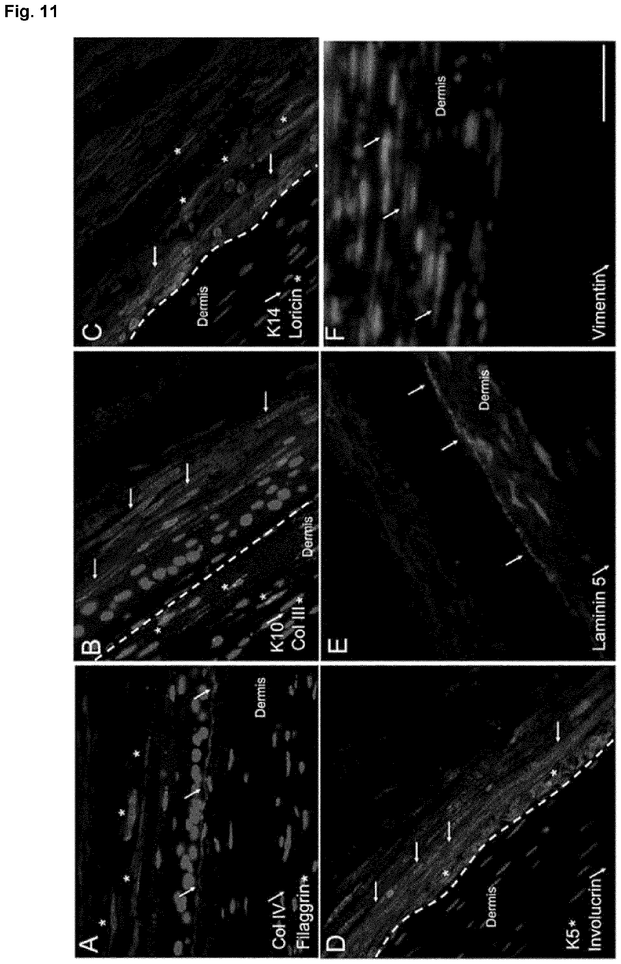

**Fig. 11:** Organotypic skin cultures at a final fibrin concentration of 3.5 mg/mL without antifibrinolytic agent after 30 days in culture using $EM_m$ characterized through immunofluorescence staining using antibodies against dermo-epidermal markers: A) collagen IV (arrow) and Filaggrin (star); B) K10 (arrow) and collagen III (star); C) K14 (arrow) and Loricrin (star); D) Involucrin (arrow) and K5 (star); E) Laminin 5 (arrow) and F) Vimentin (arrow). Scale bar: 100$\mu$m.

## GENERAL DEFINITIONS

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

It is noted that the term "about", as used herein, refers to +/- 30%, preferably +/- 20%, preferably +/- 15%, more preferably +/- 10%, of the indicated referred value.

By "skin model" is referred herein as a culture system that recreates aspects of human skin, physiology and function. Preferably, the skin model of the present invention is organotypic. By "organotypic" is referred herein as the culture of an organ that comprises one or more cell types that interact with each other in a way that resembles the natural organ. It allows the preservation of the architecture of the cultured organ, in this case the skin, and most of its cellular interactions. In an embodiment, the organotypic skin model of the present invention is a healthy human skin model, i.e., it recreates healthy human skin.

As used herein "autologous" is understood as referring to a cell preparation where the donor and the recipient are the same individual. As used herein "allogeneic" or "xenogenic" is understood as referring to a cell preparation where the donor and the recipient are not the same individual.

The term "cell population" refers to a group of cells. A cell population is heterogeneous when it comprises different groups of cells, wherein each group is differentiated from others by the presence of one or more distinguishing characteristics, such as the expression or not expression of specific markers or the presence of a different function.

The terms "%w/v" refers to the weight percentage of solute relative to the total weight or volume of the solution or dispersion, unless otherwise specified. By "v/v" or "volume per volume" is referred herein the volume percentage relative to the total volume of the solution or dispersion.

The term "clotting factor", as used herein, refers to a component, usually a protein, present in blood plasma and intervening in the chain reaction that enables coagulation. They are thirteen clotting factors, designated by Roman numbers: I: fibrinogen or fibrin; II: thrombin or prothrombin; III: tissue factor or thromboplastin; IV: calcium; V: proaccelerin; VI: inactive or zymogen factor; VII: proconvertin; VIII: A antihemophilic factor or von Willebrand factor; IX antihemophilic factor B or Christmas factor; X: Stuart-Prower factor; XI: antihemophilic factor C; XII: Hageman Factor; XIII: fibrin stabilizing factor.

The term "serum" is referred herein to blood serum and is a main component of all culture media described herein. Preferably, the blood serum is fetal serum. Unless otherwise indicated, % of serum in culture media are expressed in volume per volume (v/v).

When a cell or a population of cells is grown under "suitable culture conditions", it is referred herein as the culture conditions that allow for the viability, proliferation or growth, and retention of the phenotype of said particular cell types. The suitable culture conditions, unless indicated otherwise, are the standardized culture conditions for said particular cell type. Similarly, a suitable culture media is understood as a media that comprises the necessary elements to allow for the viability, proliferation or growth of the cell type that is grown in said suitable culture media. The skilled person knows said conditions as they are, unless otherwise indicated or specific, standard conditions in the field.

## DESCRIPTION OF THE EMBODIMENTS

Organotypic skin culture is one of the areas of tissue engineering that has developed the most in recent years because of its usefulness for *in vitro* production of skins models for pharmacological and cosmetic testing. In particular, mature skin constructs are highly valuable since they reproduce better the physiology of human skin. However, for an in vitro skin to be mature, longer period of culture are required, which usually leads to a loss of homeostasis.

Tissue homeostasis is the maintenance of the steady state within a defined tissue including control of cellular proliferation and death and control of metabolic function.This is a process that occurs naturally in the skin in vivo, but a major problem in this field is that this balance this balance is not achieved in vitro: when the skin model is cultured over long period of time (i.e., more than 15-20 days), the stratum corneum increases in thickness, the epidermis continuously differentiates and natural desquamation does not occur, leading to a loss of homeostasis that results in progressive disappearance of the granular, spinosum and finally the basal layers in favour of the stratum corneum leading to the aberrant accumulation of keratinized cells layers.

In order to solve this problem, the authors of the present invention tested two skin models based on different culture conditions: alternating differentiation and proliferation culture media (EK/EDm culture conditions) or using a maintenance culture media with only 5% of serum (EMm media). The effect of different culture conditions in epidermal differentiation and cornification were characterized through immunofluorescence, where it was found that when the cultures are alternated (EK/EDm culture conditions), there was an aberrant expression of markers K5/K14 (Fig. 8), indicating that the skin model had lost its homeostasis. However, the use of a EMm media with 5% serum resulted in a thick epidermal compartment with no extra cornification (Fig. 3C), and an increased epidermal proliferation (Fig. 4B), whereas other parameters such as the barrier (Fig. 4) or cell proliferation in the dermal compartment (Fig. 4B) integrity were not different between the two tested conditions. These results highlight the importance of using a EMm as maintenance culture media after complete epidermal differentiation.

On the other hand, it was found that supplementing hydrogels with an additional source of fibrinogen (other than that present in blood plasma) also allowed to extend organotypic skin culture time up to one month. This not only allowed a correct epidermal differentiation, but favoured skin maturation with the expression of basement membrane markers and remodelling of the dermal compartment with collagen synthesis.

In view of the above results, the present invention provides a mature *in vitro* skin organ that mimics human skin, being suitable for its use for *in vitro* testing and modelling, even when subjected to long times of culture.

In view of the above, in a **first aspect,** the present invention relates to an *in vitro* method for generating or producing a skin model, preferably an organotypic mature skin model, the method comprising the steps of:

a) providing a solution comprising a stromal cell population, blood plasma, fibrinogen, and a source of calcium; and incubating the solution until a hydrogel is formed,
b) adding to the hydrogel of step a), preferably on its surface, a cell population comprising keratinocytes and culturing said cell population and the hydrogel in a keratinocyte culture media to allow the keratinocytes to grow and expand,
c) transferring the product resulting from step b) to an air-liquid interface and culturing said product in a suitable epidermal differentiation culture media to provide an organotypic skin model with epidermal differentiation,
d) maturing the organotypic skin model with epidermal differentiation of step c) in an epidermal maintenance culture media.

The resulting organotypic skin model of step d) resembles healthy and mature human skin. Each of the steps are explained in detail below:

Step a) comprises or consists of providing a solution comprising a cell population comprising stromal cells, blood plasma, fibrinogen, and a source of calcium; and incubating the solution until a hydrogel is formed.
Step a) of the method of the first aspect comprises mixing several elements to generate a hydrogel. This hydrogel is a fibrin-based hydrogel with stromal cells embedded, and it will form the dermal base of the skin model.

The purpose of mixing fibrinogen, blood plasma, a source of calcium and stromal cells is to carry out the coagulation cascade so that a fibrin-hydrogel is formed with the cells included therein. These elements can be added simultaneously or sequentially, in any order, although it is preferred that the source of calcium, which is the element that triggers the polymerization of the fibrin, is added last. Blood plasma acts as a natural source of all the necessary components of the coagulation cascade, including thrombin that is present in the form of its precursor (inactive) prothrombin. The coagulation and, therefore, the formation of the fibrin-based hydrogel, is performed by the addition of calcium in the presence of blood plasma.

Blood plasma may be obtained by light centrifugation of the whole blood extracted by venipuncture in the presence of anticoagulants, preferably agents that chelate the calcium ion. The plasma can also be extracted by plasmapheresis. The fibrin-based hydrogel may also be formed using a plasma derivative, such as, but not limited cryoprecipitate or fibrinogen concentrate, as long as the necessary factors and enzymes to produce the transformation of fibrinogen into fibrin are provided.

Preferably, the blood plasma is bovine, porcine, or human plasma. In an embodiment, the blood plasma is autologous human plasma. The possibility that all artificial tissue components are autologous allows such tissue to be used without immunosuppression of the subject transplanted if transplanted to a patient. However, components of artificial tissue can also be allogenic origin, i.e., may come from a different person to the one who is going to receive the artificial tissue, in which case it is said that its origin is allogegenic. This opens the possibility that the artificial tissue is prepared in advance when needed urgently. Thus, in another embodiment, the human plasma is allogenic or xenogenic.

In an embodiment, the blood plasma is a solution of platelet poor plasma (PPP). Platelet poor plasma is blood plasma but with a reduced number of platelets. In an embodiment, the blood plasma is a solution of PPP characterized by comprising less than $10 \times 10^6$, less than $10 \times 10^5$, less than $10 \times 10^4$, less than $10 \times 10^3$ platelets /$\mu$L

As explained above, the blood plasma is used as a source of the necessary compounds to transform fibrinogen into fibrin. However, blood plasma can be substituted by adding each of the compounds of the clotting cascade independently. The clotting cascade that converts fibrinogen into fibrin is well known in the art and the skilled person knows how to substitute blood plasma for each of the necessary elements to be added individually. Briefly, thrombin causes breakdown of the fibrinogen molecule in low molecular weight polypeptides and in fibrin monomers. Such monomers polymerized into dimers and subsequently are joined together by covalent bonds, by factor XIII, activated by thrombin previously, and in the presence of calcium ions. The interaction between these elements promotes fibrin polymerization which results in the hydrogel formation. Preferably, the blood plasma is used as a source of clotting factor II and XIII. In a preferred embodiment, a source of clotting factor of type II and a source of clotting factor XIII is added in step a) wherein preferably said source is blood plasma. In a preferred embodiment, the source of clotting factor of type II added in step a) is thrombin or prothrombin.

Hence, the fibrinogen added in step a) will be converted into fibrin in the presence of a source of calcium and other factors provided by the blood plasma, resulting in a fibrin-based hydrogel. A fibrin-based hydrogel is a fibrin-based matrix fabricated utilizing the self-assembling properties of fibrinogen under the presence of blood plasma and calcium. In an embodiment, the hydrogel of step a) is a fibrin matrix made of crosslinked fibrin threads. By mixing together blood plasma, fibrinogen, and a source of calcium, and by leaving the mixture to stand under suitable conditions, the solution will polymerize or complete gelation and acquire hydrogel consistency.

Step a) comprises providing a solution comprising fibrinogen. It is known that blood plasma, *per se,* already comprises fibrinogen. However, for the skin model of the present invention to be resistant to contraction and shrinkage processes that alter its properties, a higher concentration of fibrinogen than that present in blood plasma is preferably added. Therefore, an important step of step a) is supplementing the fibrinogen already present in the blood plasma with an additional source of fibrinogen. As a result, the solution of step a) has a concentration of fibrinogen that is superior to the average concentration of fibrinogen present in blood plasma. In a preferred embodiment, the final concentration of fibrinogen in the solution of step a) is between 1 and 10 mg/mL. In a more preferred embodiment, the final concentration of fibrinogen in the solution of step a) is between 2 and 5 mg/mL, most preferably between 1.2 and 4.5 mg/ml, even most preferably between 3-4 mg/ml, most preferably 3.5 mg/ml. However, lower or higher concentrations could also be used. In an embodiment, the fibrinogen is autologous. However, it is preferred that the fibrinogen is allogenic or xenogenic origin. The concentration of fibrinogen that needs to be added can be calculated depending on the final amount of fibrin that is desired.

In order to better control the total amount of fibrin in the hydrogel, the blood plasma may be diluted so as to adjust the concentration of fibrinogen present in the blood plasma to a known range. In an embodiment, the blood plasma is diluted to comprise between 1-5 mg/ml of fibrinogen, preferably between 1-2 mg/ml, most preferably about 1.2 mg/ml of fibrinogen. The fibrinogen comprised in blood plasma or in the solution of blood plasma is thus supplemented with exogenous fibrinogen to provide the hydrogel of step a). Preferably, the exogenous fibrinogen added in step a) is a fibrinogen that does not come from the blood plasma, but from another source. Fibrinogen sources are known in the art and can be obtained from commercially available sources. Thus, in a preferred embodiment, step a) comprises or consists of providing a solution comprising stromal cells, blood plasma, fibrinogen, and a source of calcium; and incubating said solution until a hydrogel is formed, wherein the fibrinogen added to the solution is present in an amount superior to the average concentration of fibrinogen present in the blood plasma.

Step a) comprises providing a solution comprising a source of calcium. The concentration of a source of calcium, or calcium salt, added in step a) should be sufficient to induce polymerization of fibrinogen in the presence of blood plasma. In a preferred embodiment, this is the last element added to the mixture of step a) to avoid premature coagulation of the blood plasma before other components, such as the stromal cells, are added. In an embodiment of this first aspect of the invention, the calcium source added in step a) is a calcium salt such as, but not limited to, calcium chloride, calcium gluconate or a combination of both. In a more preferred embodiment, the calcium salt is calcium chloride, preferably a solution of calcium chloride. In a preferred embodiment, the calcium salt is a final concentration of between 0.01% and 2% (w/v), preferably 0.1% or 0.2% (w/v), most preferably 0.08 (w/v). However, lower or higher concentrations could also be used. The skilled person in the art is able to recognise when the polymerization of fibrinogen into fibrin is happening, and thus he is able to obtain suitable concentrations of calcium for this purpose. Several techniques to monitor the polymerization of fibrinogen are known, such as turbidimetric assays.

It may happen that the fibrin hydrogel starts to be degraded. There are two types of degradation: the so-called fibrinolysis, and another caused by proteolytic activities. A way of avoiding fibrinolysis degradation, via fibrinolysis (i.e. plasminogen/plasmin system) and/or via proteolysis degradation (i.e. metalloproteinases) and thereby providing a more stable hydrogel is increasing the concentration of fibrinogen in the mixture of step a) by supplementing the fibrinogen present in blood plasma with an additional source of fibrinogen, as explained above. Alternatively, or additionally, an antifibrinolytic or protease inhibitor agent may be added to the solution of step a), wherein the antifibrinolytic agent may be, but not limited, epsilon aminocaproic acid or tranexamic acid and the inhibitor of proteolytic and esterolytic activities may be, but not limitated, aprotinin. Preferably, the antifibrinolytic agent added in step a) is tranexamic acid. Tranexamic acid is a synthetic product derived from amino acid lysine with high affinity binding sites plasminogen lysine; blocking these sites prevent plasminogen binding to the surface of activated fibrin, exerting an antifibrinolytic effect. Tranexamic acid has the advantage over other antifibrinolytic agents of animal origin, not transmitted diseases. In a further preferred embodiment, the final concentration of antifibrinolytic agent, preferably tranexamic acid, in the hydrogel of step a) is between 0.01 % and 6%, preferably 0.01 and 1%, most preferably 0.08% (w/v).

Step a) comprises providing a solution comprising a stromal cell population. These cells are added in order to better reproduce the dermal layer of the skin. The population comprising stromal cells can be autologous, allogenic or xenogenic. By "stromal cells" is referred herein as connective tissue cells of any organ, preferably from the skin dermis. Preferably, the stromal cells are fibroblasts or undifferentiated cells with the ability to differentiate into fibroblast cells. Preferably, the fibroblasts are human fibroblasts. Preferably, the fibroblasts are human dermal fibroblasts. The term "fibroblasts" used herein refers to is a resident cell type of the connective tissue. Fibroblasts synthesize fibres and maintains the extracellular matrix of tissue in many animals. These cells provide a lattice-like structure (stroma) to many different tissues and play a crucial role in wound healing, being the most common cells of connective tissue. They are derived from primitive mesenchymal and pluripotent cells. In some embodiments, the population comprising stromal cell is a population comprising human fibroblasts, preferably human dermal fibroblasts. Preferably, the population of fibroblasts comprises human non-cancerous (i.e., healthy) fibroblasts. Preferably, the fibroblasts are a primary culture of human fibroblasts. Fibroblasts can be obtained from any tissue or organ; however, it is preferred herein that the fibroblasts used are skin or dermal fibroblasts, most preferably obtained from the foreskin. Hence, it is most preferred that the fibroblasts are primary human dermal fibroblasts obtained from human foreskin. It is possible to replace the dermal fibroblasts with cells of another origin, such as mesenchymal stem cells.

In some embodiments, the cell population comprising stromal cells further comprises embryonic stem cells (ESCs), non-embryonic or adult stem cells, progenitor cells, and/or iPS cells. It is noted that, in addition or alternatively, undifferentiated cells, preferably epithelial cells, with the ability to differentiate into such cells (stromal cells, preferably fibroblasts) can also be added, such as adult stem cells.

In an embodiment, the stromal cells have been previously cultured and maintained in suitable culture conditions before being used in step a). In an embodiment, the total number of stromal cells, preferably fibroblasts, added in step a) is between 5.000-80.000 cells/ml, preferably 20.000 cells/ml of solution.

The preparation of a hydrogel according to step a) preferably comprises or consists of the steps of:

a-i) preparing a cell composition comprising previously cultured stromal cells,
a-ii) adding to the composition of a-i) fibrinogen and blood plasma, and preferably an antifibrinolytic agent,
a-iii) adding to the composition of a-ii) a source of calcium, and
a-iv) incubating the solution of step iii) until a hydrogel is formed.

Step a-i) comprises or consists of preparing a cell composition comprising previously cultured stromal cells. The stromal cells are cultured in a suitable culture media, preferably in a stromal cell culture media, preferably a fibroblast culture media. The times and conditions of culturing the stromal cells, preferably fibroblasts, are not especially limited.

Culture media to grow stromal cells, preferably fibroblasts, are known in the art. Preferably, the media is a fibroblast culture media, preferably the fibroblast culture media defined in the present invention as FBm. In an embodiment, the suitable fibroblast culture media is characterized by having a percentage of serum of more than 8%, preferably of about 10%, 11%, 12%, 13%, 14%, most preferably 10% (v/v). The percentage of serum is calculated based on the total volume of the media. For example, a 10% of serum corresponds to 10 ml of serum comprised in a total volume of 100 ml. In a preferred embodiment, the suitable culture media is a fibroblast culture media (FBm), comprising Dulbecco's modified Eagle's medium (DMEM), and at least 8%, 9%, preferably 10%, 12%, 14%, 16% (v/v) serum, preferably FBS, and optionally, antibiotics and/or antifungal agents.

Steps a-i and a-ii) can be performed sequentially or simultaneously. Once steps a-i) to a-iii) are performed, it is recommended to homogenize the solution to provide a homogeneous mixture before step a-iv).

Step a-iv) of the method of the first aspect or any of its embodiments comprises or consists of allowing the mixture formed by the fibrinogen, a source of calcium, a cell population comprising stromal cells, and blood plasma polymerize or gelate to form a fibrin-derived hydrogel. By "incubating the mixture of solution until a hydrogel is formed" means allowing the resulting solution of step iii) to polymerize, thereby forming a fibrin-based hydrogel. This is done by creating the suitable conditions to allow the mixture of the above mentioned components to form chemical crosslinks that result in the polymerization or gelation of the fibrin-based hydrogel. Cross-link is a bond which links one polymer chain to other. Cross linking changes a liquid polymer into 'solid' or 'gel' by restricting the ability of movement. When polymer chains are linked together by cross-links, they lose some of their ability to move as individual polymer chains. A liquid polymer (where the chains are freely flowing) can be turned into a 'solid' or 'gel' by cross-linking the chains together. Cross linking increases the molecular mass of a polymer, which in this case is a fibrin polymer. The polymerization of the hydrogel can be assessed by naked eye, when it is observed that there is not liquid in the container.

Since a cell population comprising stromal cells, preferably fibroblasts, are also added in the solution of step a), it is desirable that the incubation of the solution until a hydrogel is formed is carried out under suitable culture conditions that allow the viability of said stromal cells, so that they can form a functional dermis layer. Thus, in an embodiment, the incubation to form the hydrogel is performed at temperatures of between 20-40°C, preferably at 34-37°C. In an embodiment, the incubation is performed at conditions at which stromal cells grow, e.g., 37°C in an atmosphere containing 5% $CO_2$ and 40% relative humidity.

It is also noted that, since the cell population comprising stromal cells, preferably fibroblasts, is added in the mixture of step a), once the polymerization of the polymer solution is carried out, said cell population will be embedded in the resulting hydrogel of step a). It is thus desirable, as explained above, that the solution of step a) is a homogeneous solution, in order to avoid uneven distribution of the stromal cells in the matrix of the hydrogel. Similarly, it is also advantageous that the conditions provided for the solution to polymerizate and form the hydrogel are suitable conditions that allow the polymerization in short period of times, to avoid cell sedimentation in the bottom of the solution. The time of fibrin polymerization mostly depends on the temperature, being 30-37°C desirable as it provides a fast polymerization of the hydrogel where the stromal cells will be evenly distributed throughout the matrix.

In an embodiment, the resulting hydrogel of step a) is incubated in a suitable keratinocyte culture media, in preparation for step b). Preferably, the keratinocyte culture media is the media defined in the present invention as "keratinocyte culture media (EKm)". Preferably the keratinocyte culture media has at least or about 8%, 9%, preferably 10%, 11%, 12%, 13%, 14% or 15% (v/v) of serum. In an embodiment the EKm media comprises a 3:1 mixture of Dulbecco's modified Eagle medium (DMEM) with HAM'S 12, about than 8% (v/v) of serum, choleric toxin, insulin, hydrocortisone and epithelial growth factor. Most preferably, the EKm media comprises a 3:1 mixture of Dulbecco's modified Eagle medium (DMEM), HAM'S F12, at least 8%, 9%, preferably 10%, 11%, 12%, 13%, 14% or 15% (v/v) or more serum, preferably of HyClone FetalClone II serum, 0.1 nM choleric toxin, 2 nM T3, 5 mg/mL insulin, 0.4 mg/mL hydrocortisone, and 10 ng/mL epithelial growth factor (EGF).

The hydrogels produced can be stored and maintained during several days at 37°C in a 5% $CO_2$ oven, completely covered in a suitable culture media for the stromal cells to grow, until the hydrogel is used for keratinocyte culture in step b). In the usual conditions of cultivation, these gels remain stable and adhered to the base and the walls of the culture flask without retractions or volume losses. Hence, a possible further step a-iv) may be culturing the hydrogel comprising stromal cells, preferably fibroblasts, completely covered in a suitable culture media for the stromal cells to grow and expand.

In a preferred embodiment of the first aspect of the invention, the skin model may further comprise at least one of the components selected from a list consisting of alginate, gelatin from several origins (fish, bovine, porcine skin), fibrin or a source of fibrin from several origins, intraarticular sodium hyaluronate, silk fibroin and sericine, xanthan, genipin, agar, agarose, chitosan, nanocellulose, proteoglycans (dermatan sulphate, hyaluronic acid, keratin, chondroitin sulphate, keratan sulphate), elastin, and other collagen types such as collagen I, II, III y IV, or their methacryloyl variants, or any combination thereof. These components, if added, are preferably be added in step a).

In an embodiment, step a) is performed during at least 12 hours, preferably 24, 36, or 48 hours before continuing with step b).

Step b) comprises or consists of adding to the product of step a), preferably on its surface, a cell population comprising keratinocytes and culturing the hydrogel in a keratinocyte culture media to allow the keratinocytes to grow.

On the hydrogel formed after step a), preferably on its surface, keratinocytes can be seeded at low density and cultivated for a sufficient amount of time to obtain pre-confluency or total cell confluency, thereby forming a stratified epithelium. The dermal layer formed by the fibrin-hydrogel comprising stromal cells, preferably fibroblasts, will allow the rapid growth of keratinocytes. In the interior of the hydrogel, the fibroblasts grow rapidly and are able to enhance the growth of the keratinocytes, even when the fibroblasts are at an extremely low initial concentration. In the hydrogel described herein, the stromal cells, preferably fibroblasts, grow by secreting substances that turn them into cells that direct and stimulate the growth of keratinocytes, which will then form the epidermis layer of the skin.

In an embodiment of the first aspect of the invention or of any of its embodiments, the cell population comprising keratinocytes added in step b) are epidermal keratinocytes, preferably from human origin. These cells can be cultured in very different ways, but it is preferred that they are cultured as a monolayer in the presence of feeder cells previous their use in step b). Said feeder cells are preferably irradiated cells that have lost their proliferation capacity but are capable of supporting the growth of keratinocytes previous their addition to the hydrogel of step a), In an embodiment, the keratinocytes have been previously cultured, expanded, and/or maintained in suitable culture conditions before being used in step b). In an embodiment, the total number of keratinocytes cells added in step a) is between 12.000-1.500.000 cells/ml, preferably $1 \times 10^6$ cells/ml.

In an embodiment, the keratinocytes are primary cells or cells directly derived from a patient or taken directly from living tissue. Thus, the population comprising keratinocytes may be autologous. In another preferred embodiment, the population comprising keratinocytes are allogenic or have a xenogenic origin. Keratinocytes can be obtained from different epithelia; however, it is preferred herein that the keratinocytes used are skin keratinocytes. In an embodiment, the keratinocytes are primary epidermal keratinocytes obtained from foreskin, preferably from human foreskin. In an embodiment, the keratinocytes are healthy, i.e., non-cancerous, keratinocytes.

In another embodiment, the population comprising keratinocytes is a population comprising keratinocytes derived from an established line that has not been directly or freshly isolated from a patient. Thus, in this case, the keratinocytes are immortalized keratinocytes.

In some other embodiments, the cell population comprising keratinocytes is a heterogeneous population comprising other epithelial cells, preferably cancerous and/or non-cancerous epithelial cells. In some embodiments, the cell population comprising keratinocyte cells further comprises embryonic stem cells (ESCs), non-embryonic or adult stem cells, progenitor cells, and/or iPS cells. It is noted that, in addition or alternatively, undifferentiated cells, preferably epithelial cells, with the ability to differentiate into such cells (keratinocytes cells) can also be added, such as adult stem cells.

The cell populations described herein (steps a) and b) of the method of the first aspect or any of its embodiments) can be obtained by different methods described in the art and may depend on the particular cell type in question. Some of these methods are, for example, but not limited to biopsy, mechanical processing, enzymatic treatment (eg, without limitation, trypsin or collagenase type I), centrifugation, erythrocyte lysis, filtration, culture media or media that favoring selective proliferation of the cell type or immunocytometry. While the cell populations are prepared to be seeded in the hydrogel, they are maintained in a suitable culture media, the culture media in which are can be partially or completely replaced by new media replacement ingredients depleted and eliminate metabolites and catabolites potentially harmful. Preferably, the cells proliferate on the surface and/or embedded in the resulting product of step a). Preferably, the stromal cells proliferate embedded in the hydrogel, while the keratinocytes proliferate on the surface of the hydrogel.

In the context of the present invention, it is to be understood that any cell populations described herein is preferably a homogeneous or substantially pure population or a mixed cell population enriched in the specific cell population (stromal cells, fibroblasts, keratinocytes). By "homogeneous" or "substantially pure population" is referred as to at least about 75%, at least about 80%, at least about 85%, at least about 90%, at least about 95%, at least about 98%, at least about 99% or 100% of the cells in the composition are the cells defined herein (stromal cells, fibroblasts, keratinocytes). The cell populations are added in a suitable media forming a cell suspension including pharmaceutically acceptable liquid media.

Preferably, the cells added in step b) are added on the surface of the hydrogel of step a). Preferably, the cell population comprising keratinocytes are added on the surface of the hydrogel of step a), but eventually grow fully or partially inside of the fibrin-based hydrogel. In an embodiment, after adding the cells in step b), the hydrogel and the cells added to it (stromal cells and keratynocites) are cultured in a keratinocyte culture media under suitable conditions to allow the keratinocytes to grow and expand. By "grow and expand" is referred herein that the keratinocytes can multiply and cover the surface of the hydrogel, thereby forming a biomimetic epidermal layer of the skin model. By growing the keratinocytes under suitable culture conditions, they exhibit an "in vitro" behaviour similar to that presented "in vivo" in human skin. They adhere, migrate and grow so that, from a few cells sown in step b), they cover the entire surface of the hydrogel and form a stratified epithelium. The final result is that from a small initial number of sown cells, a superior one, stratified epithelial cells and a lower one constituted by an extracellular matrix densely populated with stromal cells, preferably fibroblasts.

In a preferred embodiment, the keratinocytes are cultured in the keratinocyte culture media until at least 80%, 85%, 90%, 95%, 96%, 97%, 98%, 99%, or 100% cell confluency, wherein the cell confluency is measured with respect to the surface of the hydrogel. Cell confluence can be assessed by a skilled person by several means, such as by eye inspection. In the context of the present invention, cell confluence refers to the percentage of the surface of the hydrogel that is covered by keratinocyte cells that are adhered to it. For example, 50 percent confluence means that about half of the surface of the gel is covered, while 100 percent confluence means the surface is completely covered by the cells, and no more room is left for the cells to grow as a monolayer.

Suitable culture conditions to grow the keratinocytes and for their proper differentiation are known in the art. A preferred keratinocyte culture media is the media defined in the present invention as "keratinocyte culture media (EKm)", which was defined above.

Thus, in a preferred embodiment, step b) comprising culturing the hydrogel and the cells comprised in it in a keratinocyte culture media to allow the keratinocytes to grow and expand over the surface of the hydrogel until at least 80%, preferably 90%, confluency is reached, and wherein the keratinocyte culture media is characterized by comprising at least 5%, 6%, 7%, 8%, 9%, preferably 10%, 11%, 12%, 13%, 14% or 15% of serum (v/v).

Preferably, step b) is carried out until a keratinocyte cell confluency of at least 90% is reached in a keratinocyte culture media comprising at least 8%, preferably at least 10%, of serum. In an embodiment, step b) is performed during at least 24, 36, preferably 48, 60 or 72 hours until the desired confluency is reached. Once the desired % of keratinocyte cell confluency is reached, step c) is executed.

Step c) comprises or consists of transferring the product resulting from step b) to an air-liquid interface and culturing said product in a suitable epidermal differentiation culture media to provide an organotypic skin model with epidermal differentiation.

In a preferred embodiment, steps a) and b) are carried out with the hydrogel completely submerged in the suitable culture media (preferably, fibroblast culture media in step a) and keratinocyte culture media in step b)). However, at the end of step b), i.e., when the keratinocyte cells reach a certain threshold of confluency, as explained above, the product resulting from step b) is transferred to an in an air-liquid interface system.

By "air-liquid interface system" is referred herein to the culture of cells such that their basal membrane is in contact with, or submerged in, liquid and their apical membrane is in contact with air. Advantageously, the keratinocytes consequently demonstrate apical-basal polarity in their differentiation resulting in the development of functional keratinised surfaces as seen in vivo. Therefore, in step c), the epithelial surface (i.e., keratinocytes) is exposed to air to promote proper stratification and epithelial maturation of the cells, maintaining the matrix comprising the cells of step a) submerged in culture. Therefore, in a preferred embodiment, the method of the invention, comprises a step in which the resulting skin model is exposed, at least partially, to air.

In an embodiment, the hydrogel is moved to an air-liquid interface, wherein part of the hydrogel is submerged in a suitable epidermal differentiation culture media. The suitable epidermal differentiation culture media is a media that allows the keratinocytes to stop multiplying and become differentiated. In an embodiment, the suitable epidermal differentiation culture is characterized by having a low percentage of serum, to promote keratinocyte differentiation over their growth. In an embodiment, the suitable epidermal differentiation culture is characterized by having a percentage of serum of 1% or less than 1%, preferably less than 0.8%, less than 0.6%, most preferably about 0.5% (v/v) of serum.

Preferably, the epidermal differentiation culture media is the media defined herein as EDm. The EDm media comprises or consists of 3:1 mixture of Dulbecco's modified Eagle medium (DMEM), HAM'S F12, optionally penicillin/streptomycin (P/S), below 1%, most preferably about 0.5% (v/v), of serum, choleric toxin, T3, insulin, hydrocortisone, epithelial growth factor (EGF) and ascorbic acid. In most preferred embodiment, the epidermal differentiation culture media comprises or consists of 3:1 mixture of Dulbecco's modified Eagle medium (DMEM, HAM'S F12, 1% penicillin/streptomycin (P/S), 0.5% of HyClone FetalClone II serum, 0.1 nM choleric toxin, 2 nM T3, 5 mg mL-1 insulin, 0.4 mg mL-1 hydrocortisone, and 10 ng mL-1 epithelial growth factor (EGF) and $50\mu$g/mL ascorbic acid.

In an embodiment, step c) is carried out until the skin model is characterized by having an epidermal differentiation, i.e., until the keratinocytes comprised in the hydrogel are differentiated. When keratinocytes are differentiated, they express at least one, preferably all, of the following proteins: Keratin 5 (K5), Keratin 14 (K14), Keratin 10 (K10), Loricrin, Filaggrin and/or Involucrin. Please note that Keratin 5 and Keratin 14 (K14) are usually polymerized together to form the filaments of the basal layer, and consequently they are expressed together. Thus, Keratin 5 and Keratin 14 are used indistinctly to characterize de basal layer. By "epidermal differentiation" is referred herein as the expression by keratinocytes that form the epidermal layer of at least one, preferably a combination, of the following proteins: K5, K14, K10, Loricrin, Filaggrin and/or Involucrin. Additionally, "epidermal differentiation" may be also characterized by the lack of basement membrane and the lack of collagen in the dermis layer, which are characteristic of more mature skins. The basement membrane is defined below and its definition applies herein and thorough the whole document. Thus, in an embodiment, step c) is carried out until the keratinocytes present a differentiation phenotype, wherein the differentiation phenotype is characterized by the presence or the expression by the keratinocytes that form the epidermal layer of at least one, preferably a combination, of the following proteins: K5, K14, K10, Loricrin, Filaggrin and/or Involucrin, and wherein differentiation phenotype is further characterized by the lack of collagen in the dermis layer and the lack of a basement membrane. In an embodiment, step c) is performed during at least 10, 11, 12, 13, 14, preferably 15, 16, 17, 18, 19, or 20 days.

Step d) comprises or consists of maturing the organotypic skin model with epidermal differentiation of step c) in an epidermal maintenance culture media

Once the epidermis is differentiated, the skin model is matured in an epidermal maintenance culture media, thereby providing a matured organotypic skin model. This may be done for long periods of time, such as 20, 22, 24, 26, 28, 30, 32, 34, 36, 38, or 40 days. This is possible due to the percentage of serum in the epidermal maintenance culture media, which helps maintaining organ's homeostasis, thereby avoiding terminal differentiation and excessive cornification, while allowing proper maturation of the skin model. The percentage of serum in the maintaining culture media is lower than the serum of the keratinocyte culture media of step b), but higher than the epidermal differentiation culture media of step c), thereby maintaining organ's homeostasis or equilibrium between the skin layers and allowing culture for long period of time. In an embodiment, the epidermal maintenance culture media is characterized by having a percentage of serum of equal or less than 8% (v/v), but higher than 1% (v/v). Preferably, the epidermal maintenance culture media is characterized by having a percentage of serum of about 5% (v/v), preferably between 4-6%, most preferably 5% (v/v).

In a preferred embodiment, the epidermal maintenance culture media is the media defined herein as EMm. EMm preferably comprises or consists of 3:1 mixture of Dulbecco's modified Eagle medium (DMEM), HAM'S F12, optionally 1% penicillin/streptomycin (P/S), equal or less than 8% (v/v) of serum, choleric toxin, T3, insulin, hydrocortisone, epithelial growth factor (EGF) and ascorbic acid. In a preferred embodiment, the epidermal maintenance culture media comprises or consists of 3:1 mixture of Dulbecco's modified Eagle medium (DMEM), HAM'S F12, optionally 1% penicillin/streptomycin (P/S), 5% of HyClone FetalClone II serum, 0.1 nM choleric toxin, 2 nM T3, 5 mg mL-1 insulin, 0.4 mg mL-1 hydrocortisone, and 10 ng mL-1 epithelial growth factor (EGF) and $50\mu$g/mL ascorbic acid. In step e), the skin model is maintained in an air-liquid interface and is ready to be used. Uses of the skin model are further defined below.

As can be seen from the above steps, the percentage of serum is key to be able to obtain the skin model of the invention. In particular, it is important that steps a) and b) comprise the use of a culture media with high percentage of serum, such as about 10% (v/v), so that the cells can grow and reach confluency, but the % of serum is decreased in step c), preferably to less than 1% (v/v), to allow cells to stop growing and differentiate. The % of serum is then kept in step d) at about 5% (v/v), so that the skin model, which is now a differentiated skin model, can be maintained for long period of times, maintaining tissue homeostasis without incurring to terminal differentiation and cornification.

The term "maturing the skin model" refers to the culture conditions carried out as defined herein to arrive to a mature skin model, which is the result of step d). By "mature skin model" is referred herein as an organotypic skin culture comprising or consisting of:

- an epidermal compartment, wherein the epidermal compartment is in the top or the upper part of the organotypic skin culture and it comprises keratinocytes and hyaluronic acid,

- a basement membrane, wherein said basement membrane or layer is located below the epidermal compartment, and where it is characterized by being a membrane comprising collagen, preferably collagen IV, and laminin 5,
- a dermal compartment, wherein the dermal compartment is located in the base of the organotypic skin culture, and it comprises fibroblasts and collagen I and III.

In a preferred embodiment, the epidermal compartment is characterized by comprising hyaluronic acid in the epidermal intercellular space, and by comprising, from the bottom to the top, the following layers:

- a basal epidermal layer comprising cells, preferably keratinocytes, expressing keratin 5 and 14;
- a suprabasal (or spinous) epidermal layer comprising cells, preferably keratinocytes, expressing keratin 10 and involucrin,
- a granular epidermal layer comprising cells, preferably keratinocytes, expressing loricrin, and
- a cornified epidermal layer or stratum corneum comprising cells, preferably corneocytes, and filaggrin.

By "corneocytes" is referred herein as keratinocytes terminally differentiated that have lost their nucleus which are strongly anchored to one another by keratin-filaggrin attachments, resulting in flattened squames characteristic of the stratum corneum. The stratum corneum is thus characterized by the presence of filaggrin protein and corneocytes.

In a preferred embodiment, the basement membrane or layer is located between the dermal and the epidermal compartment and is mainly formed of Collagen IV and Laminin 5. The basement membrane or layer serves as the base for the keratinocytes of the epidermis layer. Preferably, the basement membrane or layer does not comprise cells.

In a preferred embodiment, the dermal compartment is located below the basement membrane or layer and is characterized by comprising fibroblasts, expressing collagen I and III. In a preferred embodiment, the dermal compartment is further characterized by comprising a matrix wherein the cells, preferably fibroblasts, are not stratified, i.e., they are not forming layers.

In an embodiment, the steps a) to d) are performed maintaining the hydrogel on a support. Supports which can be used are, for example, but not limited to tissue culture plates or porous cell culture inserts, transwell, etc. Preferably, such supports will be sterile prior use.

The time of culturing of step c) and d) is not limiting and it depends on the desired maturity degree of the skin model. If a skin model imitating early stages of epidermal differentiation is desired, then the process could be performed until step c), as step d) is a maturation step. However, if a skin model imitating late-stage skin maturation is desired, the method should comprise steps a) to d), wherein step d) is preferably carried out for more than 15 days, preferably up to 30 days.

The following are preferred embodiments of the present invention:
A method for the *in vitro* production of an organotypic skin model, the method comprising the steps of:

a) providing a solution comprising a cell population comprising stromal cells, blood plasma, fibrinogen, and a source of calcium; and incubating said solution until a hydrogel is formed,
b) adding to the hydrogel of step a), preferably on its surface, a cell population comprising keratinocytes, and culturing the hydrogel in a keratinocyte culture media to allow the keratinocytes to grow until at least 90%, preferably at least 95%, of confluency is reached, wherein the keratinocyte culture media is characterized by having a percentage of serum of more than 8% (v/v),
c) transferring the product resulting from step b) to an air-liquid interface and culturing said product in a suitable epidermal differentiation culture media to provide an organotypic skin model with epidermal differentiation, wherein the epidermal differentiation culture is characterized by having a percentage of serum of less than 1%, preferably of 0.5% (v/v), and
d) maturing the organotypic skin model with epidermal differentiation of step c) in an epidermal maintenance culture media, wherein the epidermal maintenance culture media is characterized by having a percentage of serum of between 4-6% (v/v).

A method for the *in vitro* production of an organotypic skin model, the method comprising the steps of:

a) providing a solution comprising a cell population comprising stromal cells, blood plasma, fibrinogen, and a source of calcium; and incubating said solution until a hydrogel is formed,
b) adding to the hydrogel of step a), preferably on its surface, a cell population comprising keratinocytes, and culturing the hydrogel in a keratinocyte culture media to allow the keratinocytes to grow until at least 90%, preferably at least 95%, of confluency is reached, wherein the keratinocyte culture media is characterized by having a percentage of serum of 10% (v/v),
c) transferring the product resulting from step b) to an air-liquid interface and culturing said product in a suitable epidermal differentiation culture media to provide an organotypic skin model with epidermal differentiation, wherein epidermal differentiation is characterized by the expression by the keratinocytes of at least one, preferably all, of K5, K14, K10, Loricrin, Filaggrin and/or Involucrin, and wherein the epidermal differentiation culture is characterized by having a percentage of serum of 0.5% (v/v), and

d) maturing the organotypic skin model with epidermal differentiation of step c) in an epidermal maintenance culture media, wherein the epidermal maintenance culture media is characterized by having a percentage of serum of 5% (v/v).

A method for the *in vitro* production of an organotypic skin model, the method comprising the steps of:

a) providing a solution comprising a cell population comprising stromal cells, blood plasma, fibrinogen, and a source of calcium; and incubating the solution until a hydrogel is formed,

b) adding to the hydrogel of step a), preferably on its surface, a cell population comprising keratinocytes, and culturing the hydrogel in a keratinocyte culture media to allow the keratinocytes to grow until at least 90%, preferably at least 95%, of confluency is reached, wherein the keratinocyte culture media is characterized by having a percentage of serum of 10% (v/v),

c) transferring the product resulting from step b) to an air-liquid interface and culturing said product in a suitable epidermal differentiation culture media to provide an organotypic skin model with epidermal differentiation, wherein epidermal differentiation is characterized by the expression by the keratinocytes of at least one, preferably all, of K5, K14, K10, Loricrin, filaggrin and/or involucrin, and wherein the epidermal differentiation culture is characterized by having a percentage of serum of 0.5% (v/v), and

d) maturing the organotypic skin model with epidermal differentiation of step c) in an epidermal maintenance culture media, wherein the epidermal maintenance culture media is characterized by having a percentage of serum of 5% (v/v),

wherein preferably step a) is carried out for about 24h, step b) is carried out for about 48h, step c) is carried out for about 15 days, and step d) is carried out for about 25-30 days.

A method for the *in vitro* production of an organotypic skin model, the method comprising the steps of:

a) providing a solution comprising a cell population comprising stromal cells, blood plasma, fibrinogen, and a source of calcium; and incubating the solution until a hydrogel is formed,

b) adding to the hydrogel of step a), preferably on its surface, a cell population comprising keratinocytes, and culturing the hydrogel in a keratinocyte culture media to allow the keratinocytes to grow until at least 90%, preferably at least 95%, of confluency is reached, wherein the keratinocyte culture media is characterized by having a percentage of serum of 10% (v/v),

c) transferring the product resulting from step b) to an air-liquid interface and culturing said product in a suitable epidermal differentiation culture media to provide an organotypic skin model with epidermal differentiation, wherein epidermal differentiation is characterized by the expression by the keratinocytes of at least one, preferably all, of K5, K14, K10, Loricrin, filaggrin and/or involucrin, and wherein the epidermal differentiation culture is characterized by having a percentage of serum of 0.5% (v/v), and

d) maturing the organotypic skin model with epidermal differentiation of step c) in an epidermal maintenance culture media to provide a mature skin model, wherein the epidermal maintenance culture media is characterized by having a percentage of serum of 5% (v/v), and wherein the mature skin model is characterized by comprising or consisting of, from top to the bottom:

- an epidermal compartment, wherein the epidermal compartment is in the top or the upper part of the organotypic skin culture and it comprises hyaluronic acid and keratinocytes expressing keratin 5 and14,
- a basement membrane, wherein said basement membrane is located below the epidermal compartment, and wherein it is characterized by being a membrane comprising collagen, preferably collagen IV, and laminin 5,
- a dermal compartment, wherein the dermal compartment is located in the base of the organotypic skin culture, and it comprises fibroblasts expressing collagen I and III,

wherein preferably step a) is carried out for about 24h, step b) is carried out for about 48h, step c) is carried out for about 15 days, and step d) is carried out for about 25-30 days.

A method for the *in vitro* production of an organotypic skin model, the method comprising the steps of:

a) providing a solution comprising a cell population comprising fibroblasts, blood plasma, fibrinogen, and a source of calcium; and incubating said solution in a suitable keratinocyte culture media until a hydrogel is formed,

b) adding to the hydrogel of step a), preferably on its surface, a cell population comprising keratinocytes, and culturing the hydrogel in a keratinocyte culture media to allow the keratinocytes to grow until at least 90%, preferably at least 95%, of confluency is reached, wherein the keratinocyte culture media is characterized by having a percentage of serum of 10% (v/v),

c) transferring the product resulting from step b) to an air-liquid interface and culturing said product in a suitable epidermal differentiation culture media to provide an organotypic skin model with epidermal differentiation, wherein epidermal differentiation is characterized by the expression by the keratinocytes of at least one, preferably all, of K5, K14, K10, loricrin, filaggrin and/or involucrin, and wherein the epidermal differentiation culture is characterized by having a percentage of serum of 0.5% (v/v), and

d) maturing the organotypic skin model with epidermal differentiation of step c) in an epidermal maintenance culture media to provide a mature skin model, wherein the epidermal maintenance culture media is characterized by having a percentage of serum of 5% (v/v), and wherein the mature skin model is characterized by comprising or consisting of, from top to the bottom:

- an <u>epidermal compartment</u>, wherein the epidermal compartment is in the top or the upper part of the organotypic skin culture and it comprises hyaluronic acid and keratinocytes expressing keratin 5 and 14, and wherein said epidermal compartment further comprises the following layers, from top to bottom:

  ◦ a <u>cornified epidermal layer</u> or stratum corneum comprising cells, preferably corneocytes, and filaggrin.
  ◦ a <u>granular epidermal layer</u> comprising cells, preferably keratinocytes, expressing loricrin,
  ◦ a <u>suprabasal (or spinous) epidermal</u> layer comprising cells, preferably keratinocytes, expressing keratin 10 and involucrin, and
  ◦ a <u>basal epidermal epidermal layer</u> comprising cells, preferably keratinocytes, expressing keratin 5 and 14;

- a <u>basement membrane</u>, wherein said basement membrane is located below the epidermal compartment, and wherein it is characterized by being a membrane comprising collagen, preferably collagen IV, and laminin 5,
- a <u>dermal compartment</u>, wherein the dermal compartment is located in the base of the organotypic skin culture, and it comprises fibroblasts expressing collagen I and III,

wherein preferably step a) is carried out for about 24h, step b) is carried out for about 48h, step c) is carried out for about 15 days, and step d) is carried out for about 25-30 days.

In a second aspect, the present invention provides an organotypic skin model obtained or obtainable from the method defined in the first aspect or any of its embodiments.

In a third aspect, the present invention relates to *in vitro* uses of the organotypic skin model of the invention. Said uses include the testing of test agents such as but not limited to therapeutics, cosmetics, compounds or biologically active xenobiotic agents, on skin cell function and permeability. The term "xenobiotic agent" is herein given a broad definition and includes not only compounds but also gaseous agents. Typically, xenobiotic agent encompasses pharmaceutically active agents used in human and veterinary medicine and human cosmetics. In yet a further preferred embodiment of the invention, said test agent can contact the cell culture by adding it to said cell culture media. Alternatively, said test agent can contact the cell culture by adding it to the apical surface of said organotypic model. Advantageously, this permits delivery of test agents, including vapours, gases and dry air-borne powders, in addition to soluble test-agents, this is much more representative of events that occur in-vivo wherein the skin epithelium is one of the first lines of defence to a variety of different agents.

In a preferred method of the invention the organotypic model is contacted with at least one therapeutic, cosmetic, compound, textile or xenobiotic agent.

In an embodiment, the uses include pharmacological development assays, chemical or pollutants toxicity assays, screening of medical and/or cosmetic drugs, testing mechanical irritations through textiles, basic study of the fundamental biology of skin, and/or the processes that mediate skin ageing or skin disease.

According to an aspect of the invention there is provided an organotypic skin model according to the invention for use in monitoring the ageing of skin, wherein the use includes monitoring the structural and functional properties of the skin model stratified organotypic skin model as a measure of skin age.

In an embodiment, the skin model of the present invention is used for the high throughput screening of test agents comprising the steps:

i) providing the skin model of the invention following the method of the first aspect;
ii) contacting the skin model with a plurality of agents to be tested;
iii) collecting activity data obtained following (ii) above;
iv) converting the collected data into a data analysable form; and optionally providing an output for the analysed data,

wherein preferably the agents to be tested include therapeutic, cosmetic, compound, textile or xenobiotic agents.

The invention is described below by the following examples, which must be considered as merely illustrative and in no case limiting of the scope of the present invention.

## EXAMPLES

### EXAMPLE 1: SKIN MODEL with antifibrinolytic agent

#### Materials and Methods

##### 1. Cell growth and differentiation culture media

Fibroblast culture media (FBm) - human dermal fibroblasts (hFBs) were cultured using Dulbecco's modified Eagle's medium (DMEM, GIBCOTM-BRL) containing 10% FBS and 1% (P/S).

Keratinocyte culture media (EKm) - human epidermal keratinocytes (hKCs) were cultured in a 3:1 mixture of Dulbecco's modified Eagle medium (DMEM) (GIBCOTM-BRL), HAM'S F12 (GIBCOTM-BRL), and 1% penicillin/streptomycin (P/S), containing 10% of HyClone FetalClone II serum, 0.1 nM choleric toxin, 2 nM T3, 5 mg mL-1 insulin, 0.4 mg mL-1 hydrocortisone, and 10 ng mL-1 epithelial growth factor (EGF) (Sigma, St Louis, MO, USA).

Epidermal differentiation culture media (EDm) - epidermal differentiation was induced using a cell culture media composed of 3:1 mixture of Dulbecco's modified Eagle medium (DMEM) (GIBCOTM-BRL), HAM'S F12 (GIBCOTM-BRL), and 1% penicillin/streptomycin (P/S), containing 0.5% of HyClone FetalClone II serum, 0.1 nM choleric toxin, 2 nM T3, 5 mg mL-1 insulin, 0.4 mg mL-1 hydrocortisone, and 10 ng mL-1 epithelial growth factor (EGF) and 50μg/mL ascorbic acid. (Sigma, St Louis, MO, USA)

Epidermal maintenance culture media (EMm) - once the epidermis is differentiated, organotypic cultures were maintained using a cell culture media composed of 3:1 mixture of Dulbecco's modified Eagle medium (DMEM) (GIBCOTM-BRL), HAM'S F12 (GIBCOTM-BRL), and 1% penicillin/streptomycin (P/S), containing 5% of HyClone FetalClone II serum, 0.1 nM choleric toxin, 2 nM T3, 5 mg mL-1 insulin, 0.4 mg mL-1 hydrocortisone, and 10 ng mL-1 epithelial growth factor (EGF) and 50μg/mL ascorbic acid. (Sigma, St Louis, MO, USA)

##### 2. Primary hKCs and hFBs Culture

For the dermal compartment, primary dermal fibroblasts from human foreskin (hFBs) whereas for the epidermal compartment, primary epidermal keratinocytes from human foreskin (hEK) were purchased from PromoCell. hFBs were cultured using Dulbecco's modified Eagle's medium (DMEM, GIBCOTM-BRL) containing 10% FBS and 1% (P/S), later referred as fibroblast culture media (FBm). hKCs were cultured according to the previously described methods (1) and then modified by our laboratory (2,3). Epidermal cells cannot grow in a conventional 2D-culture monolayer, and therefore they must be co-cultured with a feeder layer of lethally irradiated embryonic fibroblast from mouse 3T3-J2. The growing media for hKCs was a 3:1 mixture of Dulbecco's modified Eagle medium (DMEM) (GIBCOTM-BRL), HAM'S F12 (GIBCOTM-BRL), and 1% penicillin/streptomycin (P/S), containing 10% of fetal calf serum (FCS), 0.1 nM choleric toxin, 2 nM T3, 5 mg mL-1 insulin, 0.4 mg mL-1 hydrocortisone, and 10 ng mL-1 epithelial growth factor (EGF) (Sigma, St Louis, MO, USA), later referred as epidermal keratinocyte culture media (EKm).

##### 3. Organotypic Skin Cultures

Organotypic skin cultures were made at final fibrin concentrations of 2.5, 3.5, and 4.5 mg/mL. With that purpose, human blood plasma fibrin concentration was fixed at 1.2mg/mL and supplemented with pure commercial fibrinogen to reach the final concentrations. Plasma hydrogels were prepared inside transwells (Fisher Scientific, Frederick, MA, USA) to generate dermo-epidermal skin constructs in vitro. Frozen human blood plasma with a low platelet content was obtained from a blood bank in Spain (Banco de Sangre del Centro Comunitario de Transfusión del Principado de Asturias (CCST)) and treated according to the standards of the American Association of Blood Banks (4). The organotypic skin cultures were generated using plasma of known fibrinogen concentrations from different batches to ensure reproducibility. Plasma-derived hydrogels were prepared following the protocol previously described by Meana et al. (2). Briefly, calculations were performed to attain the volume of plasma needed to obtain hydrogels with a final fibrinogen concentration of 1.2 mg/mL and supplemented with pure commercial fibrinogen from human plasma (Sigma Aldrich, Burlington, Vermont, US) to get a final concentration of 2.5, 3.5 and 4.5 mg/mL keeping plasma always at a fixed concentration of 1.2 mg/mL (Figure 1A). The stock solution of fibrinogen was 20 mg/mL in sodium chloride (0,9% w/v NaCl). Antifibrinolytic agent tranexamic acid (AmchaFibrin 500 mg, Meda Pharma SL, Madrid, Spain) was added at a final concentration of 0.008% w/v. Finally, in order to trigger the coagulation cascade, sterile filtered calcium chloride (CaCl2 1%, Sigma Aldrich, Burlington, Vermont, USA) was added to a final concentration of 0.08% (w/v). The mixture was adjusted to the final desired volume by adding the corresponding volume of sodium chloride (NaCl 0.9% (w/v), Sigma Aldrich, Burlington, Vermont, USA). hFBs were incorporated at 20,000 cells/mL diluted in EKm. Once homogenized, the solution was deposited within the insert of the culture plates and then incubated for 1 hour at 37°C and 5% $CO_2$ (Figure 1B). After hydrogel formation, EKm was added on top and in the bottom of the insert and incubated for 24 hours at 37°C and 5% $CO_2$ and 60% humidity to remove the excess of $CaCl_2$.

hEKs were seeded on top of the plasma hydrogels at a density of $1 \times 10^6$ cells using EKm. Keratinocytes were left to sink and attach to the hydrogel surface for 48 h. After that, cultures were inspected under the microscope to check cell confluency (Figure 1C). If hKE monolayer was not confluent, cultures were incubated until confluency was reached. Transwells were then transferred to deepwell plates (Fisher Scientific, Frederick, MA, USA) to allow keratinocyte differentiation at the air-liquid interface. The hydrogel surface was conscientiously dried with the vacuum for correct epidermal differentiation. Finally, epidermal differentiation culture medium (EDm) was added into the deepwell plates, and constructs were incubated at 37°C and 5% $CO_2$. For correct cell nourishment and differentiation, culture medium must reach the bottom but not touch the walls of the insert (Figure 1D). To analyze the effect of cell culture media in epidermal differentiation, three different culture conditions were tested: I) Culture for 30 days using EDm; after 15 days in culture, the epidermal compartment is differentiated, point at which II) Exchange EKm and EDm each 2-3 days or III) culture for extra 15 days using EMm (Figure 1E). Culture medium was exchanged every 2-3 days and organotypic cultures are cultured for a minimum of 15 extra days.

Skin differentiation and maturation was stopped at different timepoints (days 10, 15, 20, and 30, with time zero being when the organotypic skin culture is moved to the air-liquid interface) to analyze the skin structure. At each timepoint, a dermo-epidermal equivalent of each fibrin concentration was fixed in a formalin-free tissue fixer (Sigma Aldrich, Burlington, Vermont, USA) for 24 h. After this, they were paraffin-embedded for histological and immunofluorescence analyses.

4. Transepithelial Electrical Resistance (TEER)

The barrier integrity of the organotypic skin cultures was analyzed after 30 days in culture by TEER measurement. Briefly, a pair of Ag/AgCl probes and a Millicell-ERS2 volt-ohm meter (Merck) was used according to the manufacturer guidelines using PBS 1X as solvent. TEER values of the models were calculated according to the following equation:

$$TEER = (Rsample - Rblank) \cdot Effective\ area$$

Rsample: resistance value of the dermo-epidermal skin equivalents (in $\Omega$).
Rblank: resistance value of a transwell without cultured cells (in $\Omega$).
Effective area: 4.2 cm2.

For each condition, 5 measurements were taken in different areas of the construct to analyze reproducibility.

5. **5-bromo-2'-deoxyuridine** (BrdU) incorporation for proliferation Analyses

To analyze cell proliferation within the organotypic skin cultures, culture media in the deep wells was replaced by new EMm with BrdU (B23151, Thermo Fisher Scientific, USA) at a final concentration of $3 \mu g/ml$ and incubated for 16 hours at 37°C and 5% $CO_2$. After that, samples were fixed using a formalin-free tissue fixer (Sigma Aldrich, Burlington, Vermont, USA) for 24 h. After this, they were paraffin-embedded for histological and immunofluorescence analyses.

6. Histological and Immunofluorescence Analyses

For the structural analyses, 5 $\mu$m thick tissue sections were stained with hematoxylin and eosin (H/E) using standard protocols (5). For the immunofluorescence analysis, 5 $\mu$m thick tissue sections were analyzed using primary specific antibodies against well-known skin markers: anti-human vimentin (1:100, monoclonal MA5-11883, Thermofisher, Frederick, MA, USA to distinguish hFB), anti-keratin 5 (1:50, polyclonal PA5-32465, Thermofisher, Frederick, MA, USA; to label hKC of the proliferative basal layer), anti-keratin 14 (1:200, monoclonal MA5-11599, Thermofisher, Frederick, MA, USA; to label hKC of the proliferative basal layer), anti-keratin 10 (1:400, monoclonal MA5-13705, Thermofisher, Frederick, MA, USA; to label suprabasal keratinocytes), anti-involucrin (1:100, monoclonal MA5-11803, Thermo Fisher Scientific, USA, to label suprabasal keratinocytes), anti-loricrin (1:100, polyclonal PA5-30583, Thermofisher, Frederick, MA, USA; to label the cornified envelope of the epidermis), anti-human filaggrin (1:100, monoclonal MA5-13440, Thermofisher, Frederick, MA, USA; to label the epidermal granular layer), anti-collagen III (1:100, polyclonal 600-401-105S, MA5-13440, Thermofisher, Frederick, MA, USA, to label the collagen synthesis in the dermis) and anti-collagen IV (1:100, monoclonal 14-9871-82, eBioscience, San Diego CA, USA, to label the basement membrane), anti-Laminin 5 (1:250, monoclonal ab78286, Abcam, Cambridge, UK, , to label the basement membrane) and Hyaluronic Acid Binding protein (1:100, AMS.HKD-BC40, AMSBIO, Cambridge, MA, USA). For primary antibody localization, tissue sections were incubated using either anti-mouse Alexa 488, anti-rabbit Alexa 555 (1:1000, A32723 and A27039, Thermofisher, Frederick, MA, USA) or Streptavidin-conjugated Alexa 488 (1:1000, S11223, Thermofisher, Frederick, MA, USA). Finally, samples were incubated for 5 min with DAPI for nuclei staining and coverslipped using DPX (06522, Sigma Aldrich, Burlington, Vermont, USA). Image acquisition was performed using an inverted microscope Leica DMi8 and an objective HC PL Fluotar 63X.

To visualize cell proliferation within the tissue, tissue sections were first deparaffined and then incubated for 1 hour at RT in 1M HCl. After that, proliferative cells were localized using anti-BrdU (1:100, monoclonal MA3-071, Thermo Fisher Scientific, USA) primary antibody. For primary antibody localization, tissue sections were incubated using anti-mouse Alexa 488 (1:1000, A32723, Thermofisher, Frederick, MA, USA).

### 7. Picrosirius red

For the histological visualization for Collagen I and III fibers, Picro Sirius Red Stain (ab150681, Abcam, Cambridge, UK) was performed following supplier's instructions. Following deparaffinization and rehydration, the sections were introduced in Picro Sirius Red Solution to completely cover the tissue and were incubated for 60 min. They were quickly rinsed in 2 changes of Acetic Acid Solution (0.5%) and rinsed again in absolute alcohol. Finally, sections were cleared in Citrus Clearing Solvent (8301, Richard-Allan Scientific, Thermo Fisher Kalamazoo, MI, USA) and mounted in DPX (06522, Sigma Aldrich, Burlington, Vermont, USA).

Samples were visualized using a direct microscope to study tissue structure using a light polarizer (Leica Microsystems) to differentiate the collagen fibers more easily from the background. Collagen type I (thick fibers) are shown in yellow/orange birefringence and Collagen type III (thin fibers) are observed as green birefringence. For protein quantification, images were analysed using Image J program to quantify the area of yellow/orange (for collagen I, $A_o$), green (for collagen III, $A_G$) and black/grey (for fibrin, $A_B$). The percentage of collagen in the dermal compartment was represented as:

$$\%Collagen_I = {A_o}/{A_t}$$

$$\%Collagen_{III} = {A_G}/{A_t}$$

$$\%Collagen_T = \%Collagen_I + \%Collagen_{III}$$

Where $A_t = A_0 + A_G + A_B$ and represent the total area of the dermal compartment.

### Results

In order to analyze the biological behaviour of the different matrices, structural analysis of the organotypic skin cultures were performed. After 15 days in culture, there was a correct epidermal differentiation for all fibrin concentrations (Figure 2(B-D)), showing a cuboidal basal layer that differentiate upward with a final differentiation into the stratum corneum. These results are similar to those of the control conditions in terms of epidermal differentiation but showing a thicker dermal compartment (Figure 2A). This fact led us to conclude that increasing fibrin concentration provide us with a more stable dermal compartment that allow to expand organotypic skin cultures lifetime, which is crucial to ensure complete skin maturation.

One of the main goals of this work is to extend culture times to ensure correct skin maturation. For that reason, organotypic skin cultures were maintained in culture up to 30 days using different culture conditions. Structural analysis of these construct shows the presence of a very thick stratum corneum for when EDm was used (Figure 3A). Although this thickening could be due to the absence of desquamation of the terminal skin layers, it has associated the narrowing of the epidermal compartment. In this context, cornification of the constructs is solely caused by the demise of the epidermal layers, which evidences a lack of homeostasis in the cultures. This phenomenon has been previously reported in the literature, pointing out the need to carefully analyze and characterize different culture conditions to avoid excesive epidermal terminal differentiation and cornification (6-8). Tissue homeostasis is based on a balance between cell differentiation and cell proliferation, with many clinical conditions associated to a disruption of this equilibrium.

Epidermal differentiation requires low serum culture media to promote cell differentiation over proliferation. However, once the epidermal compartment has been formed, maintaining these culture conditions for longer times could lead to an imbalance between these two processes. For that reason, two different culture conditions were tested: alternate differentiation and proliferation culture media (EK/EDm) and the use of a culture media with only a 5% of serum (EMm). Structural analysis of the organotypic cultures showed no differences in the cornification process for EK/EDm condition (Figure 3B), whereas the use of EMm maintain tissue homeostasis with a thick epidermal compartment with no extra-cornification (Figure 3C). Despite this result, no significant differences were found in the evaluation of the barrier integrity (TEER) between different culture conditions (Figure 4).

Additionally, the effect that different culture conditions has on cell proliferation was analyzed in terms of BrdU incorporation (Figure 5). Whereas no differences were found in cell proliferation of the dermal compartment, epidermal proliferation drastically increased for EMm culture condition (Figure 5B). Immunofluorescence reveled that cells withing the basal layer are those with positive expression of BrdU, being crucial to maintain tissue renewal capacity (Figure 5A). Despite this promising result, proliferation values showed a wide distribution, that may be explained because different regions of the tissue were analyzed and no all of them were at the same stage.

To characterize whether extended culture conditions provide with more mature organotypic skin constructs, dermal compartment remodelling was characterized in terms of collagen synthesis. Picrosirius staining showed that collagen synthesis started after 15 days in culture, and drastically increased when the constructs are cultured for 30 days (Figure 6(A-B)). Quantitative analysis of collagen composition of the dermal compartment revealed that after 30 days in culture the presence of collagen ranges from a 7- to a 36% depending on the culture conditions, reaching the maximum value when EMm was used (Figure 6C). Additionally, this analysis showed that for all the cases the amount of collagen III (green) is always predominant over the presence of collagen I (yellow/orange). These results characterize collagen synthesis as a process in which collagen III is firstly synthetized by fibroblast (Figure 6B) and later replaced by collagen I (9,10).

Conscientious analysis of specific dermo-epidermal skin markers through immunofluorescence showed positive expression of K14 (Figure 7C) and K5 (Figure 7D) in the basal layer, K10 (Figure 7B) and Involucrin (Figure 7D) in the suprabasal layer, Loricrin (Figure 7C) in the granular layer and Filaggrin (Figure 7A) in the *stratum corneum* and Hyaluronic Acid in the intercellular space (Figure 8). Moreover, extended culture times allowed the synthesis and maturation of the basement membrane, with positive expression of Col IV (Figure 7A) and Laminin 5 (Figure 7E), present in the lamina densa region of the basement membrane. Regarding the dermal compartment, fibroblast showed positive expression of vimentin (Figure 7F) and collagen III (Figure 7B).

The effect of different culture condition onto epidermal differentiation and cornification were characterized through immunofluorescence. For EK/EDm culture conditions, there was positive expression of K5 and K14 not only in the basal layer but in the suprabasal (Figure 9B). Moreover, in addition to the excessive cornification, K5 is expressed by a-nucleated cells within the *stratum corneum* (Figure 9(A, C)). This aberrant expression of K5/K14 proteins suggested a problem in the differentiation process when both culture medias were alternated. The condition at which cell proliferation is promoted (EKm) drastically changed to a differentiation state (EDm) within a short period of time, having a negative effect onto correct epidermal differentiation.

Supplementing fibrin hydrogels with extra commercial fibrinogen allowed us to extend organotypic skin culture time up to one month. This not only allows a correct epidermal differentiation but favors skin maturation with the expression of basement membrane markers (11-13) and remodeling of the dermal compartment with collagen synthesis. Despite the 36% of the dermal compartment comprises collagen, is far from the 98% present in mature skin. This is a promising result as longer culture times would allow more mature skin constructs. In addition to all that, the analysis of different culture conditions led us to solve a widely current problem, the epidermal thickening, caused solely by stratum corneum increase that disrupts tissue homeostasis and did not allow for extended culture times. All these results led us to conclude that the suitable culture conditions to obtain more mature organotypic skin cultures is the use of fibrin hydrogels at a final fibrin concentration of 3.5mg/mL and the use of EMm after complete epidermal differentiation. This methodology provides with a new product that better resembles human skin, being suitable for its used for in vitro testing and modelling.

## References

1. Rheinwald JG, Green H. Serial cultivation of strains of human epidermal keratinocytes: the formation of keratinizing colonies from single cells. Cell. 1975 Nov;6(3):331-43.
2. Meana A, Iglesias J, Del Rio M, Larcher F, Madrigal B, Fresno MF, et al. Large surface of cultured human epithelium obtained on a dermal matrix based on live fibroblast-containing fibrin gels. Burns. 1998 Nov;24(7):621-30.

3. Del Rio M, Larcher F, Serrano F, Meana A, Muñoz M, Garcia M, et al. A preclinical model for the analysis of genetically modified human skin in vivo. Hum Gene Ther. 2002 May;13(8):959-68.

4. FAUST RJ. Technical Manual of the American Association of Blood Banks. Anesthesiology [Internet]. 1986 Aug 1;65(2):242. Available from: https://doi.org/10.1097/00000542-198608000-00044

5. Cardiff RD, Miller CH, Munn RJ. Manual Hematoxylin and Eosin Staining of Mouse Tissue Sections. Cold Spring Harb Protoc [Internet]. 2014 Jun 1;2014(6):pdb.prot073411. Available from: http://cshprotocols.cshlp.org/content/2014/6/pdb.prot073411.abstract

6. Stark H-J, Boehnke K, Mirancea N, Willhauck MJ, Pavesio A, Fusenig NE, et al. Epidermal homeostasis in long-term scaffold-enforced skin equivalents. J Investig dermatology Symp Proc. 2006 Sep;11(1):93-105.

7. Stark H-J, Willhauck MJ, Mirancea N, Boehnke K, Nord I, Breitkreutz D, et al. Authentic fibroblast matrix in dermal equivalents normalises epidermal histogenesis and dermoepidermal junction in organotypic co-culture. Eur J Cell Biol. 2004 Dec;83(11-12):631-45.

8. Montero A, Quilez C, Valencia L, Girón P, Jorcano JL, Velasco D. Effect of Fibrin Concentration on the In Vitro Production of Dermo-Epidermal Equivalents. Int J Mol Sci. 2021 Jun;22(13).

9. Epstein EHJ, Munderloh NH. Human skin collagen. Presence of type I and type III at all levels of the dermis. J Biol Chem. 1978 Mar;253(5):1336-7.

10. Cheng W, Yan-Hua R, Fang-Gang N, Guo-An Z. The content and ratio of type I and III collagen in skin differ with age and injury. African J Biotechnol. 2011;10(13):2524-9.

11. Couchman JR, Gibson WT. Expression of basement membrane components through morphological changes in the hair growth cycle. Dev Biol. 1985 Apr;108(2):290-8.

12. Sage H. Collagens of basement membranes. J Invest Dermatol. 1982 Jul;79 Suppl 1:51s-59s.

13. Ockleford CD, McCracken SA, Rimmington LA, Hubbard ARD, Bright NA, Cockcroft N, et al. Type VII collagen associated with the basement membrane of amniotic epithelium forms giant anchoring rivets which penetrate a massive lamina reticularis. Placenta. 2013 Sep;34(9):727-37.

**EXAMPLE 2: SKIN MODEL without antifibrinolytic agent**

In order to elucidate if the skin model of the present invention would still present its features when the antifibrinolytic agent is not added, the authors repeated the protocol to generate the skin model as defined in Example 1, but without adding tranexamic acid. The skin model generated is represented in Figu. 10 and Fig. 11. It can be observed that the properties and characteristic of maturity and differentiation were maintained despite of the lack of tranexamic acid. These results show that tranexamic acid is not essential to produce the skin model of the present invention.

**Claims**

1. A method for the *in vitro* production of an organotypic skin model, the method comprising the steps of:

   a) providing a solution comprising stromal cells, blood plasma, fibrinogen, and a source of calcium; and incubating said solution until a hydrogel is formed,
   b) adding to the hydrogel of step a), preferably on its surface, a cell population comprising keratinocytes, and culturing the hydrogel in a keratinocyte culture media to allow the keratinocytes to grow until at least 90% of confluency is reached, wherein the keratinocyte culture media is **characterized by** having a percentage of serum of more than 8% (v/v),
   c) transferring the product resulting from step b) to an air-liquid interface and culturing said product in a epidermal differentiation culture media to provide an organotypic skin model with epidermal differentiation, wherein the epidermal differentiation culture media is **characterized by** having a percentage of serum of less than 1% (v/v), and
   d) maturing the organotypic skin model with epidermal differentiation of step c) in an epidermal maintenance culture media, wherein the epidermal maintenance culture media is **characterized by** having a percentage of serum of between 4-6% (v/v).

2. The method according claim 1, wherein the step d) is carried out until the keratinocytes are differentiated as indicated by the expression in the epidermal layer of Keratin 5, Keratin 14, Keratin 10, Collagen IV, Laminin 5, Loricrin, Filaggrin and/or Involucrin proteins.

3. The method according to claim 1 or 2, wherein the resulting matured organotypic skin model of step d) is **charac-**

**terized by** comprising:

- an epidermal compartment, wherein the epidermal compartment is in the upper part of the organotypic skin model, and it comprises keratinocytes and hyaluronic acid,
- a basement membrane, wherein said basement membrane is located below the epidermal compartment, and where it is **characterized by** being a membrane comprising collagen IV and laminin 5,
- a dermal compartment, wherein the dermal compartment is located in the base of the organotypic skin culture, and it comprises fibroblasts, collagen III and I.

4. The method according to claim 3, wherein the epidermal compartment is **characterized by** comprising, from top to bottom:

- a cornified epidermal layer or stratum corneum comprising cells, preferably corneocytes, and filaggrin,
- a granular epidermal layer comprising cells, preferably keratinocytes, expressing loricrin,
- a suprabasal (or spinous) epidermal layer comprising cells, preferably keratinocytes, expressing keratin 10 and involucrin, and
- a basal epidermal layer comprising cells, preferably keratinocytes, expressing keratin 5 and 14.

5. The method according to any of claims 1 to 4, wherein the cell population comprising fibroblasts cells added in step a) are dermal fibroblasts from human foreskin.

6. The method according to any of claims 1 to 5, wherein the cell population comprising keratinocytes cells added in step b) are epidermal keratinocytes from human foreskin.

7. The method according to any of claims 1 to 6, wherein the final concentration of fibrin in the resulting hydrogel of a) is between 1.2-4.5 mg/mL, preferably 3.5 mg/mL.

8. The method according to any of claims 1 to 7, wherein the solution of plasma has a concentration of platelets of less than $10 \times 10^5$ platelets/$\mu$L.

9. The method according to any of claims 1 to 8, wherein an antifibrinolytic agent is further added to the solution of step a).

10. The method according to any of claims 1 to 9, wherein the source of calcium added in step a) is at a final concentration of between 0.01-2%, preferably 0.08% (w/v).

11. The method according to any of claims 1 to 10, wherein the total number of fibroblasts cells added in step a) is 5.000-80.000 cells/ml, preferably 20.000 cells/ml.

12. The method according to any of claims 1 to 11, wherein the total number of keratinocytes cells added in step b) is 12.000-1.500.000 cells/ml, preferably $1 \times 10^6$ cells/ml.

13. An organotypic skin model obtained or obtainable from the method according to claims 1 to 12.

14. *In vitro* use of an organotypic skin model obtained or obtainable from the method according to claims 1 to 13 in pharmacological development assays, chemical or pollutants toxicity assays, screening of medical and/or cosmetic drugs, testing mechanical irritations through textiles, basic study of the fundamental biology of skin, and/or the processes that mediate skin ageing or skin disease.

**Fig. 1**

**Fig. 2**

**Fig. 2 (cont.)**

**Fig. 3**

**Fig. 4**

TEER values for different culture conditions

**Fig. 5**

**Fig. 6**

Fig.7

**Fig. 7 (cont.)**

**Fig.8**

**Fig. 9**

Fig. 9 (cont.)

**Fig. 10**

Fig. 11

EP 4 394 029 A1

Europäisches Patentamt

European Patent Office

Office européen des brevets

# EUROPEAN SEARCH REPORT

**Application Number**

EP 22 38 3318

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MONTERO ANDRÉS ET AL: "Hyaluronic acid–fibrin hydrogels show improved mechanical stability in dermo-epidermal skin substitutes", MATERIALS SCIENCE AND ENGINEERING C, ELSEVIER SCIENCE S.A, CH, vol. 128, 31 July 2021 (2021-07-31), XP086757746, ISSN: 0928-4931, DOI: 10.1016/J.MSEC.2021.112352 [retrieved on 2021-07-31] | 13,14 | INV. C12N5/071 ADD. A61L27/38 |
| A | * page 4 – page 5 * | 1-12 | |
| X | JP 2020 202754 A (SHISEIDO CO LTD) 24 December 2020 (2020-12-24) | 13,14 | |
| A | * claim 1 * | 1-12 | |
| X | GOPU SRIRAM ET AL: "Full-thickness human skin-on-chip with enhanced epidermal morphogenesis and barrier function", MATERIALS TODAY, vol. 21, no. 4, 1 May 2018 (2018-05-01), pages 326-340, XP055655212, AMSTERDAM, NL ISSN: 1369-7021, DOI: 10.1016/j.mattod.2017.11.002 | 13,14 | |
| A | * page 2 – page 5; figure 1 * | 1-12 | |

-/--

**TECHNICAL FIELDS SEARCHED (IPC)**

C12N
A61L

The present search report has been drawn up for all claims

| Place of search | Date of completion of the search | Examiner |
|---|---|---|
| Munich | 22 May 2023 | Paresce, Donata |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 22 38 3318

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | MEANA A ET AL: "Large surface of cultured human epithelium obtained on a dermal matrix based on live fibroblast-containing fibrin gels", BURNS, BUTTERWORTH HEINEMANN, GB, vol. 24, no. 7, 1 November 1998 (1998-11-01), pages 621-630, XP002204017, ISSN: 0305-4179, DOI: 10.1016/S0305-4179(98)00107-7 | 13,14 | |
| A | * page 1 - page 3 * | 1-12 | |
| X | LLAMES S G ET AL: "Human plasma as a dermal scaffold for the generation of a completely autologous bioengineered skin", TRANSPLANTATION, WILLIAMS AND WILKINS, GB, vol. 77, no. 3, 1 February 2004 (2004-02-01), pages 350-355, XP002428654, ISSN: 0041-1337, DOI: 10.1097/01.TP.0000112381.80964.85 | 13,14 | |
| A | * the whole document * | 1-12 | TECHNICAL FIELDS SEARCHED (IPC) |
| X | KOBER JOHANNA ET AL: "Generation of a Fibrin Based Three-Layered Skin Substitute", BIOMED RESEARCH INTERNATIONAL, vol. 2015, 1 January 2015 (2015-01-01), pages 1-8, XP093027205, ISSN: 2314-6133, DOI: 10.1155/2015/170427 Retrieved from the Internet: URL:http://downloads.hindawi.com/journals/bmri/2015/170427.pdf> | 13,14 | |
| A | * page 1 - page 3 * | 1-12 | |
| X | WO 2016/039687 A1 (UNIV SINGAPORE [SG]) 17 March 2016 (2016-03-17) | 13,14 | |
| A | * figure 8; example 7 * | 1-12 | |

-/--

The present search report has been drawn up for all claims

| Place of search | Date of completion of the search | Examiner |
|---|---|---|
| Munich | 22 May 2023 | Paresce, Donata |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 22 38 3318

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/187162 A1 (CAO TONG [SG] ET AL) 5 July 2018 (2018-07-05) | 13,14 | |
| A | * claim 30; figure 1 * | 1-12 | |

TECHNICAL FIELDS
SEARCHED    (IPC)

The present search report has been drawn up for all claims

| Place of search | Date of completion of the search | Examiner |
|---|---|---|
| Munich | 22 May 2023 | Paresce, Donata |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 38 3318

This annex lists the patent family members relating to the patent documents cited in the above-mentioned European search report.
The members are as contained in the European Patent Office EDP file on
The European Patent Office is in no way liable for these particulars which are merely given for the purpose of information.

22-05-2023

| Patent document cited in search report | | | Publication date | Patent family member(s) | | Publication date |
|---|---|---|---|---|---|---|
| JP 2020202754 | A | | 24-12-2020 | NONE | | |
| WO 2016039687 | A1 | | 17-03-2016 | NONE | | |
| US 2018187162 | A1 | | 05-07-2018 | CA | 2990590 A1 | 29-12-2016 |
| | | | | CN | 107849530 A | 27-03-2018 |
| | | | | EP | 3310903 A1 | 25-04-2018 |
| | | | | JP | 2018518970 A | 19-07-2018 |
| | | | | SG | 10201910792W A | 30-01-2020 |
| | | | | US | 2018187162 A1 | 05-07-2018 |
| | | | | WO | 2016209166 A1 | 29-12-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **RHEINWALD JG ; GREEN H.** Serial cultivation of strains of human epidermal keratinocytes: the formation of keratinizing colonies from single cells. *Cell,* November 1975, vol. 6 (3), 331-43
- **MEANA A ; IGLESIAS J ; DEL RIO M ; LARCHER F ; MADRIGAL B ; FRESNO MF et al.** Large surface of cultured human epithelium obtained on a dermal matrix based on live fibroblast-containing fibrin gels. *Burns,* November 1998, vol. 24 (7), 621-30
- **DEL RIO M ; LARCHER F ; SERRANO F ; MEANA A ; MUÑOZ M ; GARCIA M et al.** A preclinical model for the analysis of genetically modified human skin in vivo. *Hum Gene Ther.,* May 2002, vol. 13 (8), 959-68
- **FAUST RJ.** *Technical Manual of the American Association of Blood Banks. Anesthesiology [Internet].,* 01 August 1986, vol. 65 (2), 242, https://doi.org/10.1097/00000542-198608000-00044
- **CARDIFF RD ; MILLER CH ; MUNN RJ.** *Manual Hematoxylin and Eosin Staining of Mouse Tissue Sections. Cold Spring Harb Protoc [Internet].,* 01 June 2014, vol. 2014 (6, http://cshprotocols.cshlp.org/content/2014/6/pdb.prot073411.abstract
- **STARK H-J ; BOEHNKE K ; MIRANCEA N ; WILLHAUCK MJ ; PAVESIO A ; FUSENIG NE et al.** Epidermal homeostasis in long-term scaffold-enforced skin equivalents. *J Investig dermatology Symp Proc.,* September 2006, vol. 11 (1), 93-105
- **STARK H-J ; WILLHAUCK MJ ; MIRANCEA N ; BOEHNKE K ; NORD I ; BREITKREUTZ D et al.** Authentic fibroblast matrix in dermal equivalents normalises epidermal histogenesis and dermoepidermal junction in organotypic co-culture. *Eur J Cell Biol.,* December 2004, vol. 83 (11-12), 631-45
- **MONTERO A ; QUILEZ C ; VALENCIA L ; GIRÓN P ; JORCANO JL ; VELASCO D.** Effect of Fibrin Concentration on the In Vitro Production of Dermo-Epidermal Equivalents. *Int J Mol Sci.,* June 2021, vol. 22 (13
- **EPSTEIN EHJ ; MUNDERLOH NH.** Human skin collagen. Presence of type I and type III at all levels of the dermis. *J Biol Chem.,* March 1978, vol. 253 (5), 1336-7
- **CHENG W ; YAN-HUA R ; FANG-GANG N ; GUO-AN Z.** The content and ratio of type I and III collagen in skin differ with age and injury. *African J Biotechnol.,* 2011, vol. 10 (13), 2524-9
- **COUCHMAN JR ; GIBSON WT.** Expression of basement membrane components through morphological changes in the hair growth cycle. *Dev Biol.,* April 1985, vol. 108 (2), 290-8
- **SAGE H.** Collagens of basement membranes. *J Invest Dermatol,* July 1982, vol. 79 (1), 51s-59s
- **OCKLEFORD CD ; MCCRACKEN SA ; RIMMINGTON LA ; HUBBARD ARD ; BRIGHT NA ; COCKCROFT N et al.** Type VII collagen associated with the basement membrane of amniotic epithelium forms giant anchoring rivets which penetrate a massive lamina reticularis. *Placenta,* September 2013, vol. 34 (9), 727-37